(19) Europäisches Patentamt / European Patent Office / Office européen des brevets

(11) **EP 4 455 839 A1**

(12) **EUROPEAN PATENT APPLICATION**
published in accordance with Art. 153(4) EPC

(43) Date of publication:
**30.10.2024 Bulletin 2024/44**

(21) Application number: **23842327.1**

(22) Date of filing: **18.07.2023**

(51) International Patent Classification (IPC):
**G06F 3/01** *(2006.01)*

(52) Cooperative Patent Classification (CPC):
**A61B 5/00; A61B 5/11; G01D 5/20; G06F 3/01**

(86) International application number:
**PCT/CN2023/107984**

(87) International publication number:
**WO 2024/017264 (25.01.2024 Gazette 2024/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **22.07.2022 CN 202210873140
09.03.2023 PCT/CN2023/080488**

(71) Applicant: **Shenzhen Shokz Co., Ltd.
Shenzhen, Guangdong 518108 (CN)**

(72) Inventors:
• **DENG, Wenjun**
  **Shenzhen, Guangdong 518108 (CN)**
• **LIAO, Fengyun**
  **Shenzhen, Guangdong 518108 (CN)**
• **QI, Xin**
  **Shenzhen, Guangdong 518108 (CN)**

(74) Representative: **Wang, Bo
Panovision IP
Ebersberger Straße 3
85570 Markt Schwaben (DE)**

(54) **WEARABLE APPARATUS**

(57) The embodiments of the present disclosure disclose a wearable apparatus, comprising: a wearable body. A plurality of inductive sensors may be distributed on the wearable body. The plurality of inductive sensors include a plurality of inductive sensors crossing a joint contour. Each of the plurality of inductive sensors includes an inductive structure that is enclosed by a conductive wire. Each of the plurality of inductive sensors is attached to a position of the wearable body corresponding to a joint position and generates variable inductances with deformations of the joint position.

FIG. 1

## Description

### CROSS REFERENCE TO RELATED APPLICATIONS

This application claims priority to International Application No. PCT/CN2023/080488, filed on March 9, 2023, and Chinese Patent Application No. 202210873140.4, filed on July 22, 2022, the contents of each of which are incorporated herein by reference.

### TECHNICAL FIELD

The present disclosure relates to the field of wearable apparatuses, and in particular to wearable apparatus.

### BACKGROUND

Movement capture technology is an important way of human-computer interaction. The movement capture technology is widely applied to AR/VR interaction, wearable electronic apparatuses, sports training, film and television entertainment, health care, and other fields. The movement capture technology can be categorized into a mechanical manner, an acoustic manner, an electromagnetic manner, an optical manner, and an inertial navigation manner. At present, a movement capture device usually uses multi-axis inertial sensors to read movement directions and speeds of human limbs using accelerometers and gyroscopes. However, the complex movement capture device is expensive, extremely uncomfortable for the user to wear, and has large errors, complex algorithms, severe drifts, frequent calibrations, and high costs. In addition, existing movement capture devices are poor in reliability and consistency, and are easily interfered by various external factors such as temperature, sweat, and pressure, and the application scenarios are very limited.

Therefore, it is desirable to provide a wearable apparatus with a simple structure, a comfortable wearing experience, a small error, and a strong anti-interference ability to achieve accurate and stable movement capture.

### SUMMARY

One of the embodiments of the present disclosure provides a wearable apparatus, comprising: a wearable body. A plurality of inductive sensors may be distributed on the wearable body. The plurality of inductive sensors may include a plurality of inductive sensors crossing a joint contour. Each of the plurality of inductive sensors may include an inductive structure that is enclosed by a conductive wire. Each of the plurality of inductive sensors may be attached to a position of the wearable body corresponding to a joint position and may generate variable inductances with deformations of the joint position.

In some embodiments, the inductive structure may include a long axis direction and a short axis direction. The joint contour may pass through a 1/3 middle region of the inductive structure in the long axis direction.

In some embodiments, the plurality of inductive sensors may include a plurality of inductive sensors crossing an arm root contour. Each of the plurality of inductive sensors crossing the arm root contour may include a first inductive structure enclosed by a first conductive wire. The first inductive structure may be configured to generate variable inductances with movements of a shoulder joint of a user.

In some embodiments, the plurality of inductive sensors crossing the arm root contour may include: an inductive sensor disposed in front of a shoulder, an inductive sensor disposed at the rear of a shoulder, and an inductive sensor disposed under an armpit.

In some embodiments, a distance between every two inductive sensors of the plurality of inductive sensors crossing the arm root contour may be greater than 3 cm.

In some embodiments, the wearable body may further include a capacitive sensor or a resistive sensor distributed near the shoulder joint. The capacitive sensor may be configured to generate variable capacitances with movements of a shoulder joint of a user. The resistive sensor may be configured to generate variable resistances with the movements of the shoulder joint of the user.

In some embodiments, the first inductive structure may include a long axis direction and a short axis direction. The arm root contour may pass through a 1/3 middle region of the first inductive structure in the long axis direction.

In some embodiments, the plurality of inductive sensors may include a plurality of inductive sensors crossing an elbow contour. Each of the plurality of inductive sensors crossing the elbow contour may include a second inductive structure enclosed by a second conductive wire. The plurality of inductive sensors crossing the elbow contour may be configured to generate variable inductances with movements of an elbow joint of the user.

In some embodiments, the second inductive structure may include a long axis direction and a short axis direction. The elbow contour may pass through a 1/3 middle region of the second inductive structure in the long axis direction.

In some embodiments, a distance between every two inductive sensors of the plurality of inductive sensors crossing the elbow contour may be greater than 3 cm.

In some embodiments, the plurality of inductive sensors may include a plurality of inductive sensors crossing a waist contour. Each of the plurality of inductive sensors crossing the waist contour may include a third inductive structure enclosed by a third conductive wire. The plurality of inductive sensors crossing the waist contour may be symmetrically distributed with respect to a central sagittal plane of the user in a wearing state and the plurality of inductive sensors may be configured to generate variable inductances with movements of waist of the user.

In some embodiments, the third inductive structure may include a long axis direction and a short axis direction. The waist contour may pass through a 1/3 middle region of the third inductive structure in the long axis direction.

In some embodiments, a distance between every two inductive sensors of the plurality of inductive sensors crossing the waist contour may be greater than 3 cm.

In some embodiments, the plurality of inductive sensors may include a plurality of inductive sensors crossing a knee contour. The plurality of inductive sensors crossing the knee contour may be disposed on an inner side or an outer side of a knee joint of the user in a wearing state and the plurality of inductive sensors may be configured to generate variable inductances with movements of the knee joint of the user.

In some embodiments, each of the plurality of inductive sensors crossing the knee contour may include a fourth inductive structure enclosed by a fourth conductive wire. The fourth inductive structure may include a long axis direction and a short axis direction. The knee contour may pass through a 1/3 middle region of the fourth inductive structure in the long axis direction.

In some embodiments, a distance between every two inductive sensors of the plurality of inductive sensors crossing the knee contour may be greater than 3 cm.

In some embodiments, the wearable body may be further provided with a plurality of inductive sensors disposed at a hip bone position. The plurality of inductive sensors disposed at the hip bone position may be configured to generate variable inductances with movements of a hip joint of the user.

In some embodiments, the plurality of inductive sensors disposed at the hip bone position may include a left hip sensor and a right hip sensor. In a wearing state, a connection line between a highest point of a left hip bone and a highest point of a right hip bone of the user may pass through the left hip sensor and the right hip sensor, respectively.

In some embodiments, a distance between every two inductive sensors of the plurality of inductive sensors disposed at the hip bone position may be greater than 3 cm.

In some embodiments, the wearable body may further include a crotch sensor disposed under a crotch, a left front hip sensor disposed at a left front position of the hip bone, and a right front hip sensor disposed at a right front position of the hip bone.

In some embodiments, intersection points of the connection line between the highest point of the left hip bone and the highest point of the right hip bone of the user and an outer edge of the wearable body may be a first intersection point and a second intersection point, respectively. A connection line between the first intersection point and a center point of the crotch may pass through a 1/3 middle region of the left front hip sensor in the long axis direction. A connection line between the second intersection point and the center point of the crotch may pass through a 1/3 middle region of the right front hip sensor in the long axis direction.

In some embodiments, the wearable apparatus may further comprise a processing circuit. The processing circuit may be configured to generate a parameter reflecting a joint movement. Each of the plurality of inductive sensors may be connected with the processing circuit through the conductive wire.

In some embodiments, the wearable apparatus may further comprise a processing circuit and distributed readout subsystems. The distributed readout subsystems may be configured to read data from at least a portion of the plurality of inductive sensors. The processing circuit may be configured to generate the parameter reflecting the joint movement based on the data. At least the portion of the plurality of inductive sensors may transmit the data to a specific readout subsystem of the distributed readout subsystems. The distributed readout subsystems may be connected with the processing circuit for communication.

In some embodiments, the conductive wire may include an elastic and stretchable conductive yarn. The elastic and stretchable conductive yarn may be fixed by weaving.

In some embodiments, the conductive wire may form a spiral inductive structure.

In some embodiments, the spiral inductive structure may include more than one turn of the conductive wire.

In some embodiments, a thickness of the conductive wire in a direction perpendicular to a surface of an inductive pattern may not be greater than 3 mm.

In some embodiments, a surface of the conductive wire may be wrapped with at least one layer of insulating material.

In some embodiments, a resistance of the inductive structure may be less than 100 Ω.

In some embodiments, at least one of the plurality of inductive sensors may further include a substrate configured to carry the spiral inductive structure. The spiral inductive structure may include at least a first layer coil and a second layer coil. The first layer coil and the second layer coil may be disposed in layers in a direction perpendicular to the substrate. A current direction in the first layer coil and a current direction in the second layer coil may be the same.

In some embodiments, the first layer coil and the second layer coil may be respectively disposed on two sides of the substrate.

In some embodiments, a through hole may be provided in the substrate. The first layer coil and the second layer coil may be respectively formed by a same conductive wire passing through the through hole.

In some embodiments, at least one of the plurality of inductive sensors may further include a protective layer configured to package the spiral inductive structure.

Some embodiments of the present disclosure provide an inductive sensor, comprising: a spiral inductive structure formed by a conductive wire; and a substrate, configured to carry the spiral inductive structure. The spiral inductive structure may include at least a first layer coil and a second layer coil. The first layer coil and the second layer coil may be disposed in layers in a direction perpendicular to the substrate. A current direction in the first layer coil and a current direction in the second layer coil may be the same.

## BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering indicates the same structure, wherein:

FIG. 1 is a schematic structural diagram illustrating an exemplary wearable apparatus according to some embodiments of the present disclosure;
FIG. 2 is a schematic structural diagram illustrating a front side of an exemplary wearable body according to some embodiments of the present disclosure;
FIG. 3 is a schematic structural diagram illustrating a back side of an exemplary wearable body according to some embodiments of the present disclosure;
FIG. 4 is a schematic structural diagram illustrating a front side of an exemplary wearable body according to some embodiments of the present disclosure;
FIG. 5 is a schematic structural diagram illustrating a back side of an exemplary wearable body according to some embodiments of the present disclosure;
FIG. 6 is a schematic structural diagram illustrating a front side of an exemplary wearable body according to some embodiments of the present disclosure;
FIG. 7 is a schematic structural diagram illustrating a back side of an exemplary wearable body according to some embodiments of the present disclosure;
FIG. 8 is a block diagram illustrating an exemplary wearable apparatus according to some embodiments of the present disclosure;
FIG. 9 is a schematic structural diagram illustrating an exemplary readout unit according to some embodiments of the present disclosure;
FIG. 10 is a schematic structural diagram illustrating an exemplary inductive sensor according to some embodiments of the present disclosure;
FIG. 11 is a schematic diagram illustrating a top view of an exemplary inductive sensor according to some embodiments of the present disclosure;
FIG. 12 is a schematic diagram illustrating a cross section of an exemplary wearable apparatus according to some embodiments of the present disclosure;
FIG. 13 is a schematic diagram illustrating a user wearing an exemplary wearable apparatus according to some embodiments of the present disclosure; and
FIG. 14 is a schematic diagram illustrating a curve showing a variation in an inductance value of an exemplary inductive structure having a spiral inductive pattern with a bending angle of the inductive structure in a non-wearing state according to some embodiments of the present disclosure.

## DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for a person having ordinary skills in the art to apply the present disclosure to other similar scenarios in accordance with these drawings without creative labor. Unless obviously obtained from the context or the context illustrates otherwise, the same numeral in the drawings refers to the same structure or operation.

It should be understood that "system," "device," "unit," and/or "module" as used herein is a method for distinguishing different components, elements, parts, portions or assemblies of different levels. However, the words may be replaced by other expressions if other words can achieve the same purpose.

As indicated in the disclosure and claims, the terms "a," "an," and/or "the" are not specific to the singular form and may include the plural form unless the context clearly indicates an exception. Generally speaking, the terms "comprising," and "including" only suggest the inclusion of clearly identified steps and elements, and these steps and elements do not constitute an exclusive list, and the method or device may also contain other steps or elements.

The flowchart is used in the present disclosure to illustrate the operations performed by the system according to the embodiments of the present disclosure. It should be understood that the preceding or following operations are not necessarily performed in the exact order. Instead, various steps may be processed in reverse order or simultaneously. Meanwhile, other operations may be added to these procedures, or a certain step or steps may be removed from these procedures.

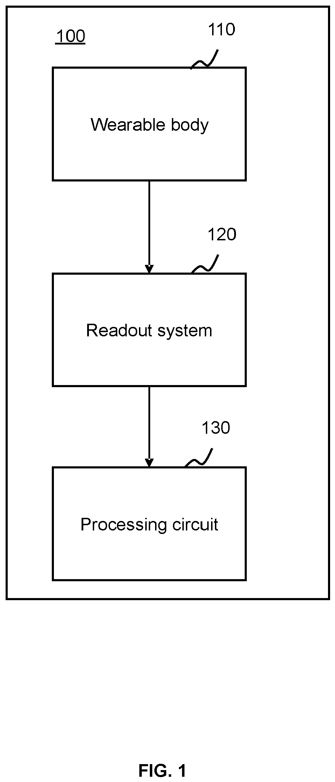

FIG. 1 is a schematic structural diagram illustrating an exemplary wearable apparatus according to some embodiments of the present disclosure.

In some embodiments, a wearable apparatus 100 may include a wearable body 110, a readout system 120, and a processing circuit 130.

The wearable body 110 refers to a component configured to cover a specific part (e.g., a joint position, an organ position, skin, etc.) of a user. For example, the wearable body 110 may be a sportswear, sports pants, a knee pad, a wrist guard, an elbow pad, a sleeve, etc., that fit closely to the body of the user. In some embodiments, the wearable body 110 may include at least one of a top wear, pants, or a jumpsuit including the top wear and the pants. It should be understood that the "top wear" in the present disclosure not only refers to clothing worn on an upper body, but also includes a part of the jumpsuit corresponding to the upper body. The present disclosure is described by taking the wearable body 110 covering the joint position of the user as an example. It can be understood that the wearable body 110 may cover any position and be configured to capture a movement of the user. In some embodiments, the joint position of the user may include a shoulder joint, an elbow joint, a wrist joint, a hip joint, a knee joint, an ankle joint, etc.

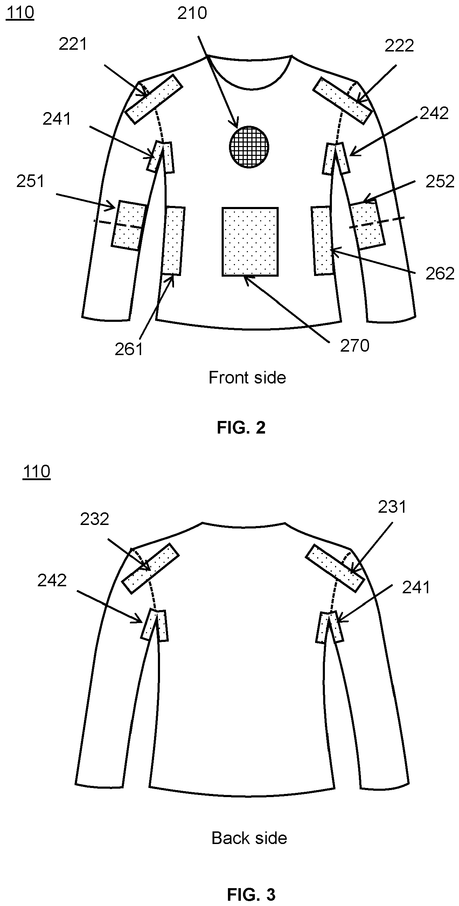

In some embodiments, a plurality of inductive sensors may be distributed on the wearable body 110. The plurality of inductive sensors may include a plurality of inductive sensors crossing a joint contour. The "joint contour" in the present disclosure refers to a circular line that characterizes the location of a joint. In the field of clothing, the joint contour provides a basis for the positioning of main structural lines of clothing. For example, the joint may include the shoulder joint, the elbow joint, the waist joint, the knee joint, etc. Accordingly, the joint contour of the shoulder joint may be an arm root contour, the joint contour of the elbow joint may be an elbow contour, the joint contour of the waist joint may be a waist contour, and the joint contour of the knee joint may be a knee contour. Descriptions regarding the arm contour, the elbow contour, the waist contour, the knee contour, etc., may be found in FIGs. 2-7 and related descriptions thereof.

Each of the plurality of inductive sensors may include an inductive structure enclosed by a conductive wire. Each of the plurality of inductive sensors may be attached to a position of the wearable body 110 corresponding to a joint position and generate variable inductances with deformations of the joint position. For example, the plurality of inductive sensors may include one of an inductive sensor disposed at the shoulder joint, an inductive sensor disposed at the elbow joint, an inductive sensor disposed at the hip joint, an inductive sensor disposed at the knee joint, an inductive sensor disposed at the wrist joint, and an inductive sensor disposed at the ankle joint, or any combination thereof. In some embodiments, the wearable body 110 may include an inductive sensor distributed at a certain part of the body, and the inductive sensor may be configured to generate a variable inductance with a deformation of the part of the body. For example, the wearable body 110 may include a plurality of inductive sensors distributed at a waist, and the plurality of inductive sensors may be configured to generate variable inductances with movements of the waist of the user. In some embodiments, the wearable body 110 may also include other types of sensors (e.g., a capacitive sensor or a resistive sensor) to generate other variable parameters (e.g., variable capacitances or resistances) with deformations of the corresponding position.

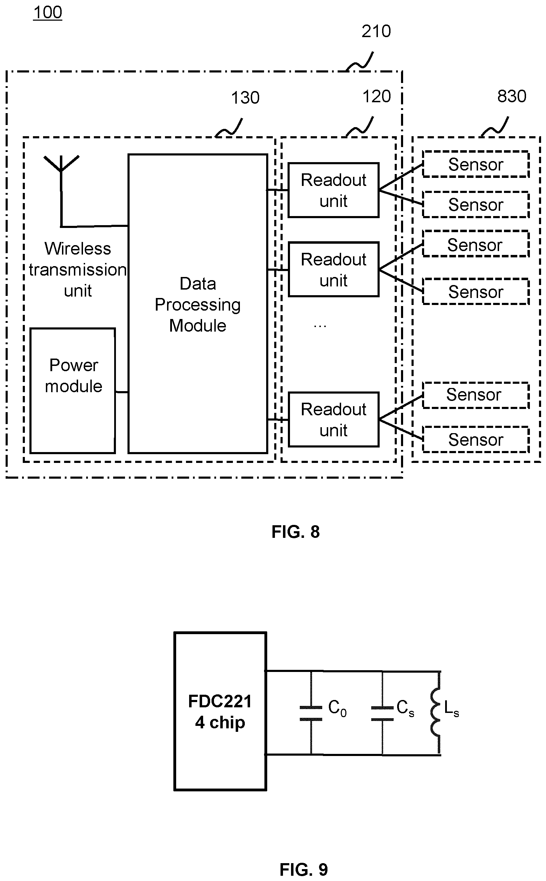

In some embodiments, the inductive structure may include a spiral inductive pattern, such as a rectangular spiral inductive pattern, a circular spiral inductive pattern, an elliptical spiral inductive pattern, etc. The inductive structure may include a long axis direction and a short axis direction. In the present disclosure, for a symmetrical spiral inductive pattern, the long axis refers to a longest axis of symmetry of the spiral inductive pattern. For an asymmetrical spiral inductive pattern, the long axis refers to a connection line between two points with a maximum distance on an outer circle of the spiral inductive pattern. For the symmetrical spiral inductive pattern, a short axis refers to a shortest axis of symmetry of the spiral inductive pattern. For the asymmetrical spiral inductive pattern, the short axis refers to a connection line between two points with a minimum distance on the outer circle of the spiral inductive pattern. In some embodiments, the long axis direction may be perpendicular to the short axis direction. In some embodiments, the long axis direction and the short axis direction of the spiral rectangular inductive pattern of each of the plurality of inductive sensors may be shown in FIG. 9.

In some embodiments, in order to improve the accuracy of capture and restoration of the movements of the joint, the joint contour may pass through a 1/2 middle region of the inductive structure in the long axis direction. The 1/2 middle region of the inductive structure in the long axis direction refers to a region of two middle parts when the inductive structure is divided into four equal parts in the long axis direction. In some embodiments, in order to further improve the accuracy of capture and restoration of the movements of the joint, the joint contour may pass through a 1/3 middle region of the inductive structure in the long axis direction. In some embodiments, in order to further improve the accuracy of capture and restoration of the movements of the joint, the joint contour may pass through a middle 1/4 region of the inductive structure in the long axis direction.

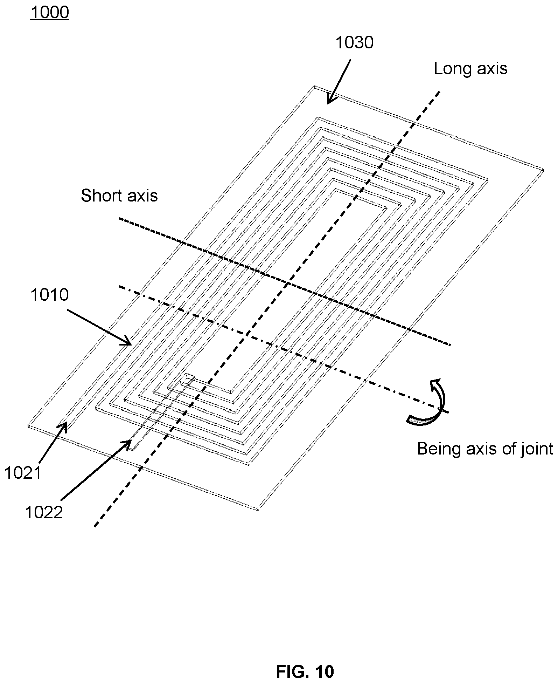

The plurality of inductive sensors refer to components that generate variable inductances with deformations. The plurality of inductive sensors may generate deformations of the plurality of inductive sensors based on deformations of a specific part of the user, and convert the deformations into electrical signals. In some embodiments, the plurality of inductive sensors may be fixed to positions of the wearable body corresponding to the joint positions by stitching, weaving, pressing, pasting, snapping, etc. Each of the plurality of inductive sensors may be enclosed by a conductive wire. Descriptions regarding the plurality of inductive sensors and the inductive structure may be found in FIGs. 10-11 and related descriptions thereof.

In some embodiments, the wearable body 110 may be configured to carry other components of the wearable apparatus. For example, the wearable body 110 may be provided with the readout system 120 and the processing circuit 130. In some embodiments, the wearable body 110 may be made of soft and close-fitting fabrics to fit closely with the joint position of the user. Exemplary fabrics may include a silk material, a cotton and linen material, a synthetic material, etc. In some embodiments, the wearable body 110 may generate corresponding deformations based on variations in movements of the human body. For example, when the user squats, the wearable body (e.g. knees of the sweatpants) may generate a deformation of extending and bending along an outer side of the knee joint and contracting and bending along an inner side of the knee joint as the user bends the knees.

The readout system 120 refers to a component configured to output electrical signals detected by a plurality of sensors (e.g., the plurality of inductive sensors). Depending on different types of the electrical signals generated by the plurality of sensors, the readout system 120 may include any one of an inductance meter, a voltammeter, an oscilloscope, a multimeter, or the like or any combination thereof. In some embodiments, the readout system 120 may be connected with the inductive structures of the plurality of sensors through a readout lead. In some embodiments, the readout system 120 may send the readout electrical signals of the plurality of sensors to the processing circuit 130.

In some embodiments, the wearable apparatus may further include the processing circuit 130 attached to the wearable body. In some embodiments, the processing circuit 130 refers to a component configured to receive and process inductance values measured by the readout system 120 or other parameters capable of characterizing variable inductances. For example, the processing circuit 130 may convert the electrical signals generated by the plurality of sensors into parameters reflecting joint movements. In some embodiments, the parameters reflecting the joint movements may include a bending angle of the joint, a flexion and extension situation of the joint, etc. The bending angle of the joint in the present disclosure may be an angle formed by skin surfaces on both sides of the joint during the deformation of the joint. In some embodiments, the electrical signals generated by the plurality of sensors and joint movement information may have a corresponding relationship. The processing circuit 130 may determine what kind of movement occurs at the corresponding joint position based on the variable electrical signals generated by the plurality of sensors. In some embodiments, the processing circuit 130 may be connected with a terminal device. For example, the processing circuit 130 may be connected with the terminal device via a network, a data line (a data interface), etc. The processing circuit 130 may generate the parameters reflecting the joint movements by processing the electrical signals (e.g., the inductance value, circuit impedance, etc.) generated by the plurality of sensors, and send the parameters to the terminal device. The terminal device may include a mobile phone, a computer, or other smart devices.

In some embodiments, the processing circuit 130 may be configured to generate the parameters reflecting the joint movements. In some embodiments, each of the plurality of inductive sensors may be connected with the processing circuit 130 through a conductive wire. In some embodiments, each of the plurality of inductive sensors may be connected with the processing circuit 130 via a wireless (e.g., Bluetooth, WIFI, etc.) connection.

It should be noted that the above description of the wearable apparatus 100 and the components thereof is only for convenience of description and does not limit the present disclosure to the scope of the embodiments. It can be understood that for those skilled in the art, after understanding the principle of the wearable apparatus 100, it is possible to arbitrarily combine the various components or form a subsystem connected to other components without deviating from this principle. For example, the readout system 120 and/or the processing circuit 130 may be an independent individual or a part of the terminal device. Such variations are within the scope of protection of the present disclosure.

With the wearable apparatus 100 described in some embodiments of the present disclosure, the joint movements of the user may be converted into the variable electrical signals, and then the parameters reflecting the joint movements may be generated, thereby accurately capturing the movements of the user. In addition, the plurality of inductive sensors may be directly integrated on the wearable body 110, making the wearable apparatus 100 comfortable and breathable and low in manufacturing cost. Furthermore, the plurality of sensors composed of the inductive structures may only respond to shape variations, so that the movement capture process may not be interfered by other factors such as temperature, pressure, sweating, etc., thereby improving the accuracy and reliability of movement capture and the reusability of the wearable apparatus 100.

In some embodiments, in order to accurately capture and restore the movements of the shoulder joint, the plurality of inductive sensors crossing the arm root contour may be distributed on the wearable body 110. Each of the plurality of inductive sensors crossing the arm root contour may include a first inductive structure enclosed by a conductive wire. The first inductive structure may be configured to generate a variable inductance with the movement of the shoulder joint of the user. Descriptions regarding the first inductive structure may be found in FIGs. 10-11 of the present disclosure.

The arm root contour refers to a reference for positioning a boundary between a body and sleeves and an armhole line of a garment on the wearable body 110 (e.g., the top wear). On the wearable body 110, the arm root contour may be embodied as the boundary between the body and the sleeves. In a wearing state, the arm root contour may be embodied as a circular line enclosing a bottom of a human arm root. In some embodiments, the arm root contour may be enclosed by at least two points. For example, a front armpit point, a back armpit point, and a shoulder endpoint may form the arm root contour by enclosing; or an armpit point, a front shoulder endpoint, and a rear shoulder endpoint may form the arm root contour by enclosing.

In some embodiments, in order to improve the accuracy of capture and restoration of the movements of the shoulder joint, for a shoulder joint (e.g., a left shoulder joint), a plurality of inductive sensors crossing the arm root contour may include at least two inductive sensors. For example, the plurality of inductive sensors crossing the arm root contour may include a front shoulder inductive sensor and a rear shoulder inductive sensor. As another example, the plurality of inductive sensors crossing the arm root contour may include the front shoulder inductive sensor and an armpit inductive sensor. As another example, the plurality of inductive sensors crossing the arm root contour may include the rear shoulder inductive sensor and the armpit inductive sensor. In the present disclosure, with a shoulder midline (a center line of the shoulder from a neck-shoulder point to a shoulder endpoint) or a central coronal plane of the human body as a dividing line, "front shoulder" refers to a part of the shoulder facing the face; and "rear shoulder" refers to a part of the shoulder facing away from the face.

In some embodiments, in order to further improve the accuracy of capture and restoration of the movements of the shoulder joint, for a shoulder joint (e.g., the left shoulder joint), the plurality of inductive sensors crossing the arm root contour may include at least three inductive sensors. For example, the plurality of inductive sensors crossing the arm root contour may include the front shoulder inductive sensor, the rear shoulder inductive sensor, and the armpit inductive sensor.

In some embodiments, other inductive sensors crossing the arm root contour may also be provided to achieve fine capture of the movements of the shoulder joint. For example, for a shoulder joint (e.g., the left shoulder joint), the plurality of inductive sensors crossing the arm root contour may include the front shoulder inductive sensor, the rear shoulder inductive sensor, the armpit inductive sensor, a latissimus dorsi region inductive sensor, a pectoralis major region inductive sensor, etc. Merely by way of example, the armpit inductive sensor may be further divided into a front armpit inductive sensor, a rear armpit inductive sensor, etc. In some embodiments, positions of the plurality of inductive sensors may be changed, and a count of plurality of inductive sensors may be decreased or increased based on actual application scenarios and conditions. For example, in case of shoulder weight (e.g., carrying a backpack), the front shoulder inductive sensor and the rear shoulder inductive sensor may be deleted. As another example, in case of large sweating under the armpit, the count of the armpit inductive sensor may be increased. Such variations all belong to the scope of the present disclosure

FIG. 2 is a schematic structural diagram illustrating a front side of an exemplary wearable body according to some embodiments of the present disclosure. FIG. 3 is a schematic structural diagram illustrating a back side of an exemplary wearable body according to some embodiments of the present disclosure. The "front side" and the "back side" refer to a side facing the face and a side facing away from the face of the wearable body 110, respectively, in a wearing state.

In some embodiments, as shown in FIG. 2 and FIG. 3, a plurality of inductive sensors crossing an arm root contour may include: an inductive sensor disposed in front of a shoulder (i.e., a front shoulder inductive sensor 221 and a front shoulder inductive sensor 222), an inductive sensor disposed at the rear of the shoulder (i.e., a rear shoulder inductive sensor 231 and a rear shoulder inductive sensor 232), and an inductive sensor disposed under an armpit (i.e., an armpit inductive sensor 241 and an armpit inductive sensor 242). The front shoulder inductive sensor 221, the rear shoulder inductive sensor 231, and the armpit inductive sensor 241 may cross a right arm root contour of the wearable body 110; and the front shoulder inductive sensor 222, the area shoulder inductive sensor 232, and the armpit inductive sensor 242 may cross a left arm root contour of the wearable body 110.

In some embodiments, the wearable body 110 may further include other types of sensors (e.g., a capacitive sensor or a resistive sensor) distributed near a shoulder joint. The capacitive sensor may be configured to generate a variable capacitance with a movement of the shoulder joint of the user. The resistive sensor may be configured to generate a variable resistance with the movement of the shoulder joint of the user. In some embodiments, the wearable body 110 may also include a flexible stretchable inductive sensor distributed near the shoulder joint. The flexible stretchable inductive sensor may be used as a part of a fabric of the wearable body 110. The flexible stretchable inductive sensor may be configured to generate a variation in a distortion of a sensor structure, material modification, an electrical property, etc., with the movement of the shoulder joint. The movement of the shoulder joint may be captured by monitoring the above variation. The movement of the same shoulder joint may be captured and restored based on different types of sensors to improve the accuracy.

In some embodiments, the first inductive structure may include a spiral inductive pattern, such as a rectangular spiral inductive pattern, a circular spiral inductive pattern, an elliptical spiral inductive pattern, etc. The first inductive structure may include a long axis direction and a short axis direction. In the present disclosure, for a symmetrical spiral inductive pattern, a long axis refers to a longest axis of symmetry of the spiral inductive pattern. For an asymmetrical spiral inductive pattern, the long axis refers to a connection line between two points with a maximum distance on an outer circle of the spiral inductive pattern. For the symmetrical spiral inductive pattern, a short axis refers to a shortest axis of symmetry of the spiral inductive pattern. For the asymmetrical spiral inductive pattern, the short axis refers to a connection line between two points with a minimum distance on the outer circle of the spiral inductive pattern. In some embodiments, the long axis direction may be perpendicular to the short axis direction. In some embodiments, the long axis direction and the short axis direction of the spiral rectangular inductive pattern of each of the plurality of inductive sensors may be shown in FIG. 9.

In some embodiments, in order to optimize sensitivities of the plurality of inductive sensors, a shape of the spiral inductive pattern may be designed symmetrically based on the long axis or the short axis. For example, the spiral inductive pattern may be a rectangle, circle, or ellipse, etc. with the long axis or the short axis as the axis of symmetry.

In some embodiments, in order to improve the accuracy of capture and restoration of the movements of the shoulder joint, the joint contour may pass through a 1/2 middle region of the first inductive structure in the long axis direction. The 1/2 middle region of the inductive structure in the long axis direction refers to a region of two middle parts when the first inductive structure is divided into four equal parts in the long axis direction. In some embodiments, in order to further improve the accuracy of capture and restoration of the movements of the shoulder joint, the arm root contour may pass through a 1/3 middle region of the first inductive structure in the long axis direction. The 1/3 middle region of the first inductive structure in the long axis direction refers to a region of a middle part when the first inductive structure is divided into three equal parts in the long axis direction. In some embodiments, in order to further improve the accuracy of capture and restoration of the movements of the shoulder joint, the arm root contour may pass through a middle 1/5 region the first inductive structure in the long axis direction. The middle 1/5 region of the first inductive structure in the long axis direction refers to a region of a middle part when the first inductive structure is divided into five equal parts in the long axis direction.

In some embodiments, in order to improve the accuracy of capture and restoration of the movements of the shoulder joint, a ratio of a distance between a center point of the first inductive structure and the arm root contour to a length of the inductive structure in the long axis direction may be less than 1/4. The center point of the first inductive structure refers to a geometric center of the first inductive structure. In some embodiments, in order to further improve the accuracy of capture and restoration of the movements of the shoulder joint, the ratio of the distance between the center point of the first inductive structure and the arm root contour to the length of the inductive structure in the long axis direction may be less than 1/6.

In some embodiments, in order to further improve the accuracy of capture and restoration of the movements of the shoulder joint, the first inductive structure may be symmetrical with respect to the arm root contour. For example, the arm root contour may equally divide the entire first inductive structure to well capture variable movements of muscles on both sides of the shoulder joint. For example, for the armpit inductive sensor, the first inductive structure of the armpit inductive sensor may be evenly folded in half, and a folding line may be the short axis of the first inductive structure. The short axis of the first inductive structure is also referred to as a bending axis of the shoulder joint and the arm root contour.

In some embodiments, in order to prevent interference between adjacent sensors, a distance between every two inductive sensors of the plurality of inductive sensors crossing the arm root contour may be greater than 3 cm. Since the working principle of the plurality of inductive sensors is realized through variable inductances, and each of the plurality of inductive sensors generates a weak magnetic field, the magnetic field of each of the plurality of inductive sensors may affect other inductive sensors. Therefore, the distance between every two inductive sensors of the plurality of inductive sensors crossing the arm root contour may be limited to avoid errors. In some embodiments, in order to further prevent interference between the adjacent sensors, the distance between every two inductive sensors of the plurality of inductive sensors crossing the arm root contour may be greater than 3.5 cm. In some embodiments, in order to further prevent interference between the adjacent sensors, the distance between every two inductive sensors of the plurality of inductive sensors crossing the arm root contour may be greater than 4 cm.

In some embodiments, in order to accurately capture and restore movements of an elbow joint, the wearable body 110 may further include a plurality of inductive sensors crossing an elbow contour. Each of the plurality of inductive sensors crossing the elbow contour may include a second inductive structure enclosed by a conductive wire. The second inductive structure may be configured to generate a variable inductance with a movement of the elbow joint of the user. Descriptions regarding the second inductive structure may be found in FIGs. 10-11 of the present disclosure.

In some embodiments, in order to accurately capture and restore the movements of the elbow joint, as shown in FIG. 2, the plurality of inductive sensors crossing the elbow contour may include an elbow inductive sensor 251 and an elbow inductive sensor 252. The elbow inductive sensor 251 may be disposed at a right elbow of the wearable body 110. The elbow inductive sensor 252 may be disposed at a left elbow of the wearable body 110. The movements of the elbow joint can be captured by using the plurality of inductive sensors crossing the elbow contour.

The elbow contour refers to a boundary between an upper elbow and a lower elbow of a sleeve of the wearable body 110. The elbow contour may reflect a circular line of an elbow between an upper arm and a forearm of the human body. In some embodiments, the elbow contour may be formed by at least two points. For example, a front elbow point and a rear elbow point may form the elbow contour.

In some embodiments, in order to further improve the accuracy of the capture of the movements of the elbow joint, the plurality of inductive sensors crossing the elbow contour may include a front elbow point inductive sensor and a rear elbow point inductive sensor (not shown in the figure). The front elbow point inductive sensor may be disposed at an inner side of the elbow joint. The rear elbow point inductive sensor may be disposed at an outer side of the elbow joint.

In some embodiments, a shape, pattern, etc., of the second inductive structure may be the same as those of the first inductive structure, which are not repeated here. In some embodiments, similar to the first inductive structure, the second inductive structure may include a long axis direction and a short axis direction. In order to improve the accuracy of capture and restoration of the movements of the shoulder joint, the elbow contour may pass through a 1/2 middle region of the second inductive structure in the long axis direction. The 1/2 middle region of the second inductive structure in the long axis direction refers to a region of two middle parts when the second inductive structure is divided into four equal parts in the long axis direction. In some embodiments, in order to further improve the accuracy of capture and restoration of the movements of the elbow joint, the elbow contour may pass through a 1/3 middle region of the second inductive structure in the long axis direction. In some embodiments, in order to further improve the accuracy of capture and restoration of the movements of the elbow joint, the elbow contour may pass through a 1/4 middle region of the second inductive structure in the long axis direction. In some embodiments, in order to further improve the accuracy of capture and restoration of the movements of the elbow joint, the elbow contour may pass through a 1/5 middle region of the second inductive structure in the long axis direction. More descriptions regarding the middle region of the second inductive structure in the long axis direction may be found in the descriptions regarding the center of the first inductive structure.

In some embodiments, in order to improve the accuracy of capture of the movements of the elbow joint, a ratio of a distance between a center point of the second inductive structure and the elbow contour to a length of the inductive structure in the long axis direction may be less than 1/4. The center point of the second inductive structure refers to a geometric center of the second inductive structure. In some embodiments, in order to further improve the accuracy of capture of the movements of the elbow joint, the ratio of the distance between the center point of the second inductive structure and the elbow contour to the length of the inductive structure in the long axis direction may be less than 1/6.

In some embodiments, in order to prevent interference between adjacent sensors, a distance between every two inductive sensors of the plurality of inductive sensors crossing the elbow contour may be greater than 3 cm. In some embodiments, in order to further prevent interference between the adjacent sensors, the distance between every two inductive sensors of the plurality of inductive sensors crossing the elbow contour may be greater than 3.5 cm. In some embodiments, in order to further prevent interference between the adjacent sensors, the distance between every two inductive sensors of the plurality of inductive sensors crossing the elbow contour may be greater than 4 cm.

In some embodiments, in order to accurately capture and restore movements of the waist, the wearable body 110 may further include a plurality of inductive sensors crossing a waist contour. Each of the plurality of inductive sensors crossing the waist contour may include a third inductive structure enclosed by a third conductive wire. The plurality of inductive sensors crossing the waist contour may be symmetrically distributed with respect to a central sagittal plane of the user in the wearing state. The plurality of inductive sensors may be configured to generate variable inductances with the movements of the waist of the user. As shown in FIG. 2, the wearable body 110 may include a waist inductive sensor 261, a waist inductive sensor 262, and an abdominal inductive sensor 270. The waist inductive sensor 261 may be disposed at a right waist of the wearable body 110. The waist inductive sensor 262 may be disposed a left waist of the wearable body 110. The abdominal inductive sensor 270 may be disposed a front abdomen of the wearable body 110. The waist inductive sensor 261 and the waist inductive sensor 262 may be symmetrically distributed with respect to the central sagittal plane of the user in the wearing state. The abdominal inductive sensor 270 may be symmetrical with respect to the central sagittal plane of the user in the wearing state.

The waist contour refers to a boundary between an upper body and a lower body of the wearable body 110. The waist contour may reflect a horizontal circular line of a thin part of the waist. In some embodiments, the waist contour may be formed by at least two points through enclosing. For example, a midpoint of a front waist, a side point of the waist, and a midpoint of a rear waist may form the waist contour.

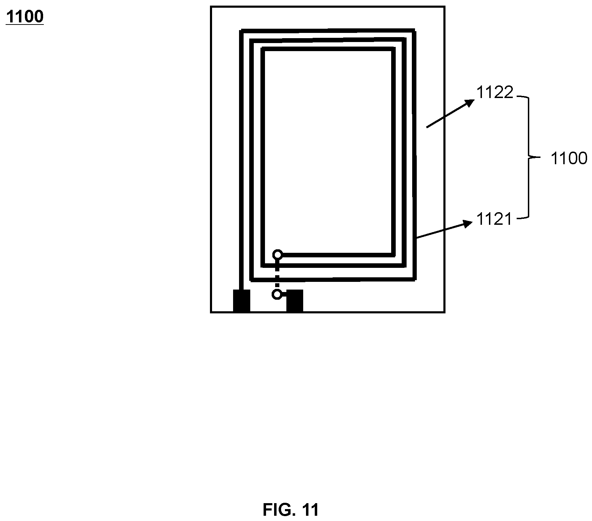

In some embodiments, a shape, a pattern, etc., of the third inductive structure may be the same as those of the first inductive structure, which are not repeated here. Descriptions regarding the third inductive structure may be found in FIG. 10 and FIG. 11 of the present disclosure.

In some embodiments, in addition to the plurality of inductive sensors crossing the waist contour, the plurality of sensors configured to capture the movements of the waist may include other sensors, such as a stretch sensor, a stress sensor, etc. By simultaneously capturing the movements of the waist with the plurality of sensors, a well capture effect of the movement can be obtained. It is understood that only one of the plurality of sensors may be configured to capture the movements of the waist while simplifying the structure.

In some embodiments, similar to the first inductive structure, the third inductive structure may include a long axis direction and a short axis direction. In order to improve the accuracy of capture and restoration of the movements of the waist, the waist contour may pass through a 1/2 middle region of the third inductive structure in the long axis direction. The 1/2 middle region of the third inductive structure in the long axis direction refers to a region of two middle parts when the third inductive structure is divided into four equal parts in the long axis direction. In some embodiments, in order to further improve the accuracy of capture and restoration of the movements of the waist, the waist contour may pass through a 1/3 middle region of the third inductive structure in the long axis direction. In some embodiments, in order to further improve the accuracy of capture and restoration of the movements of the waist, the waist contour may pass through a 1/4 middle region of the third inductive structure in the long axis direction. In some embodiments, in order to further improve the accuracy of capture and restoration of the movements of the waist, the waist contour may pass through a 1/5 middle region of the second inductive structure in the long axis direction. More descriptions regarding the middle region of the third inductive structure in the long axis direction may be found in the descriptions regarding the middle region of the first inductive structure.

In some embodiments, in order to improve the accuracy of capture and restoration of the movements of the waist, a ratio of a distance between a center point of the third inductive structure and the waist contour to a length of the inductive structure in the long axis direction may be less than 1/4. The center point of the third inductive structure refers to a geometric center of the third inductive structure. In some embodiments, in order to further improve the accuracy of capture and restoration of the movements of the waist, the ratio of the distance between the center point of the third inductive structure and the waist contour to the length of the inductive structure in the long axis direction may be less than 1/6.

In some embodiments, in order to prevent interference between adjacent sensors, a distance between every two inductive sensors of the plurality of inductive sensors crossing the waist contour may be greater than 3 cm. In some embodiments, in order to further prevent interference between the adjacent sensors, the distance between every two inductive sensors of the plurality of inductive sensors crossing the waist contour may be greater than 3.5 cm. In some embodiments, in order to further prevent interference between the adjacent sensors, the distance between every two inductive sensors of the plurality of inductive sensors crossing the waist contour may be greater than 4 cm.

In some embodiments, as shown in FIG. 2 and FIG. 3, the wearable body 110 may further include a main control circuit board 210. The main control circuit board 210 may control all the inductive sensors. More descriptions regarding the main control circuit board 210 may be found in FIG. 8 and FIG. 9 and related descriptions thereof.

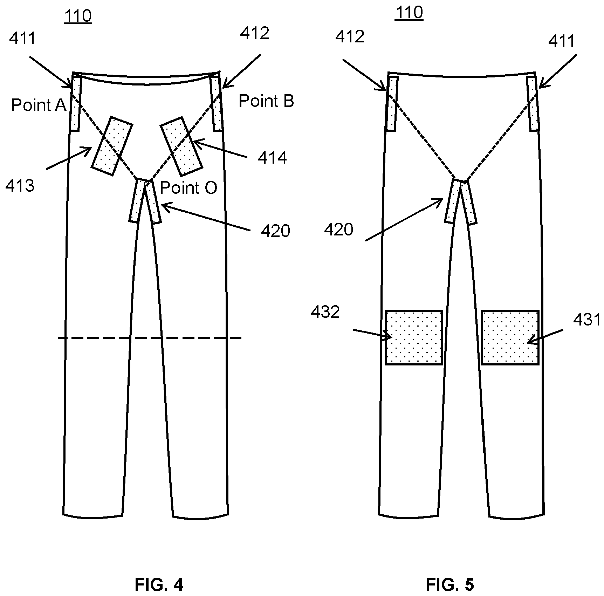

FIG. 4 is a schematic structural diagram illustrating a front side of an exemplary wearable body according to some embodiments of the present disclosure. FIG. 5 is a schematic structural diagram illustrating a back side of an exemplary wearable body according to some embodiments of the present disclosure.

In some embodiments, in order to accurately capture and restore movements of the knee joint, a plurality of inductive sensors crossing the knee contour may be distributed on the wearable body 110. The plurality of inductive sensors crossing the knee contour may be disposed at an inner side or an outer side of the knee joint of the user in the wearing state. The plurality of inductive sensors may be configured to generate variable inductance with the movements of the knee joint of the user. As shown in FIGs. 4-5, the plurality of inductive sensors crossing the knee contour may include a right knee inductive sensor 431 and a left knee inductive sensor 432.

The knee contour refers to a boundary between a calf and a thigh of a trouser leg of the wearable body 110. The knee contour may reflect a horizontal circular line of a lower limb knee through a patella point. In some embodiments, the knee contour may be formed by at least two points through enclosing. For example, an inner vertex of the knee and an outer vertex of the knee may form the knee contour.

In some embodiments, each of the plurality of inductive sensors crossing the knee contour may include a fourth inductive structure enclosed by a conductive wire. In some embodiments, a shape and a pattern of the fourth inductive structure may be the same as those of the first inductive structure, which are not repeated here.

In some embodiments, similar to the first inductive structure, the fourth inductive structure may include a long axis direction and a short axis direction. In order to improve the accuracy of capture and restoration of the movements of the knee joint, the knee contour may pass through a 1/2 middle region of the fourth inductive structure in the long axis direction. In some embodiments, in order to further improve the accuracy of capture and restoration of the movements of the knee joint, the knee contour may pass through a 1/3 middle region of the fourth inductive structure in the long axis direction. In some embodiments, in order to further improve the accuracy of capture and restoration of the movements of the knee joint, the knee contour may pass through a 1/4 middle region of the fourth inductive structure in the long axis direction. In some embodiments, in order to further improve the accuracy of capture and restoration of the movements of the knee joint, the knee contour may pass through a 1/5 middle region of the fourth inductive structure in the long axis direction. More descriptions regarding the middle region of the fourth inductive structure in the long axis direction may be found in the descriptions regarding the middle region of the first inductive structure.

In some embodiments, in order to improve the accuracy of capture and restoration of the movements of the knee joint, a ratio of a distance between a center point of the fourth inductive structure and the knee contour to a length of the inductive structure in the long axis direction may be less than 1/4. The center point of the fourth inductive structure refers to a geometric center of the fourth inductive structure. In some embodiments, in order to further improve the accuracy of capture and restoration of the movements of the knee joint, the ratio of the distance between the center point of the fourth inductive structure and the knee contour to the length of the inductive structure in the long axis direction may be less than 1/6.

In some embodiments, in order to prevent interference between adjacent sensors, a distance between every two inductive sensors of the plurality of inductive sensors crossing the knee contour may be greater than 3 cm. In some embodiments, in order to further prevent interference between the adjacent sensors, the distance between every two inductive sensors of the plurality of inductive sensors crossing the knee contour may be greater than 3.5 cm. In some embodiments, in order to further prevent interference between the adjacent sensors, the distance between every two inductive sensors of the plurality of inductive sensors crossing the knee contour may be greater than 4 cm.

In some embodiments, in order to accurately capture and restore movements of a hip bone position, a plurality of inductive sensors disposed at the hip bone position may be distributed on the wearable body 110. The plurality of inductive sensors disposed at the hip bone position may be configured to generate variable inductances with movements of a hip joint.

In some embodiments, as shown in FIGs. 4-5, the plurality of inductive sensors disposed at the hip position may include a left hip sensor 412 and a right hip sensor 411. In some embodiments, in order to improve the accuracy of movement capture at the hip bone position, the plurality of inductive sensors disposed at the hip bone position may also include a left hip sensor, a right hip sensor, etc.

In some embodiments, in order to improve the accuracy of movement capture at the hip bone position, in the wearing state, a connection line between a highest point of the left hip bone and a highest point of the right hip bone of the user may pass through the left hip sensor 412 and the right hip sensor 411, respectively. As shown in FIG. 4, intersection points of the connection line (between the highest point of the left hip bone and the highest point of the right hip bone) and an outer edge of the wearable body 110 may be a first intersection point B and a second intersection point A, respectively. A connection AB between the first intersection point B and the second intersection point A may pass through the left hip sensor 412 and the right hip sensor 411, respectively.

In some embodiments, in order to prevent interference between the adjacent sensors, a distance between every two inductive sensors of the plurality of inductive sensors disposed at the hip bone position may be greater than 3 cm. In some embodiments, in order to prevent interference between the adjacent sensors, the distance between every two inductive sensors of the plurality of inductive sensors disposed at the hip bone position may be greater than 3.5 cm. In some embodiments, in order to further prevent interference between the adjacent sensors, the distance between every two inductive sensors of the plurality of inductive sensors disposed at the hip bone position may be greater than 4 cm.

In some embodiments, since the hip joint has a relatively high degree of freedom of movement, in order to further improve the accuracy of capture and restoration of the movements of the hip joint, as shown in FIGs. 4-5, the wearable body 110 may further include a crotch inductive sensor 420 disposed under a crotch, a left front hip sensor 414 disposed at a left front position of the hip bone, and a right front hip sensor 413 disposed at a right front position of the hip bone. The left front hip refers to a pubic bone on a left side of the human body. The right front hip refers to a pubic bone on a right side of the human body.

In some embodiments, the intersection points of the connection line between the highest point of the left hip bone and the highest point of the right hip bone of the user and the outer edge of the wearable body 110 may be the first intersection point B and the second intersection point A, respectively. In some embodiments, in order to improve the accuracy of capture and restoration of the movements of the hip joint, a connection line BO between the first intersection point B and a center point O of the crotch may pass through a 1/3 middle region of the left front hip sensor 414 in the long axis direction. A connection line AO between the second intersection point A and the center point O of the crotch may pass through a 1/3 middle region of the right front hip sensor 413 in the long axis direction. As shown in FIG. 4, the connection line BO between the first intersection point B and the center point O of the crotch may intersect with the left front hip sensor 414, and the intersection point may be disposed in the 1/3 middle region of the left front hip sensor 414 in the long axis direction. Similarly, the connection line AO between the second intersection point A and the center point O of the crotch may intersect with the right front hip sensor 413, and the intersection point may be disposed in the 1/3 middle region of the right front hip sensor 413 in the long axis direction. In some embodiments, in order to further improve the accuracy of capture and restoration of the movements of the hip joint, the connection line BO between the first intersection point B and the center point O of the crotch may pass through a 1/4 middle region of the left front hip sensor 414 in the long axis direction, and the connection line AO between the second intersection point A and the center point O of the crotch may pass through a 1/4 middle region of the right front hip sensor 413 in the long axis direction. Descriptions regarding the middle region of the right front hip sensor 413 or the left front hip sensor 414 in the long axis direction may be found in the related descriptions of the middle region of the first inductive structure.

In some embodiments, the wearable body 110 may also include a left hip sensor and a right hip sensor (not shown in the figure) configured to capture movements of a hip of the user.

In some embodiments, the wearable body 110 shown in FIG.s 4-5 may also include a main control circuit board (not shown in the figures). The main control circuit board may be configured to control all the inductive sensors. Descriptions regarding the main control circuit board may be found in FIGs. 8-9 and related descriptions thereof. In some embodiments, the wearable body 110 may share the same main control circuit board 210 with the wearable body 110, or the wearable body 110 may each include the main control circuit board 210.

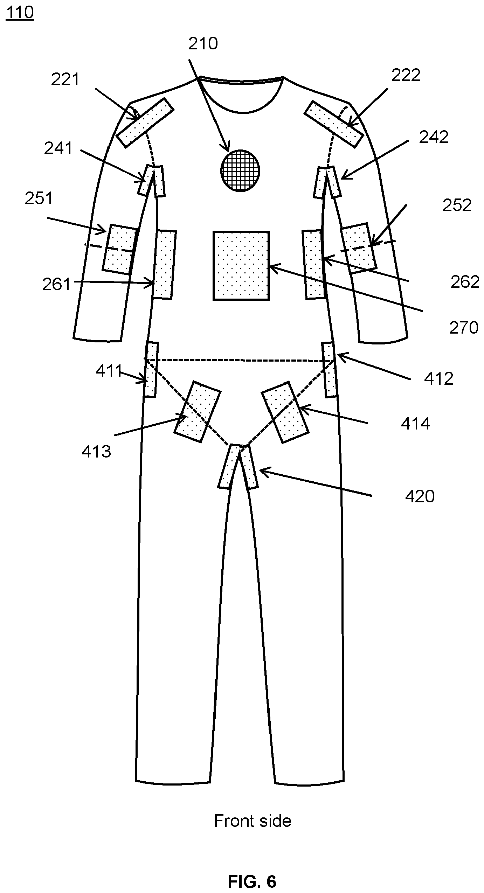

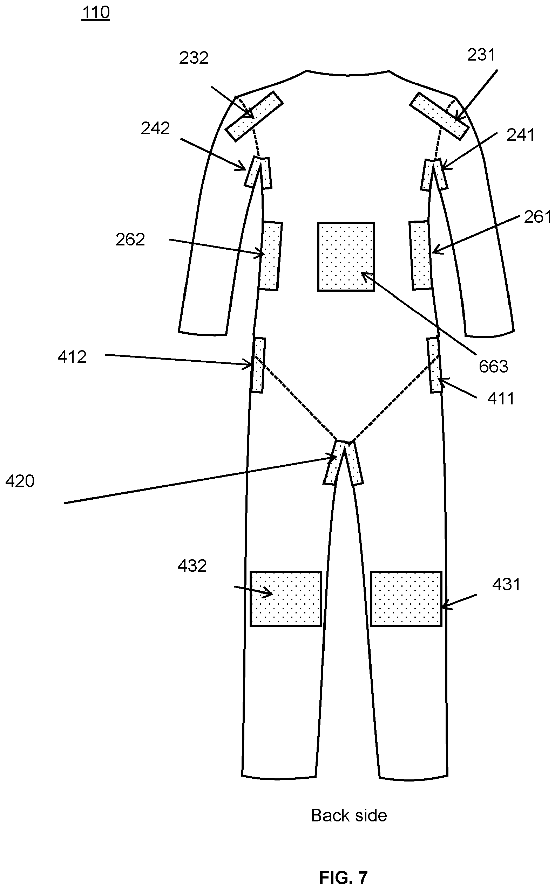

FIG. 6 is a schematic structural diagram illustrating a front side of an exemplary wearable body according to some embodiments of the present disclosure. FIG. 7 is a schematic structural diagram illustrating a back side of an exemplary wearable body according to some embodiments of the present disclosure. As shown in FIGs. 6-7, the wearable body 110 may be a jumpsuit. The wearable body 110 may include the main control circuit board 210, the front shoulder inductive sensor 221, the front shoulder inductive sensor 222, the rear shoulder inductive sensor 231, the rear shoulder inductive sensor 232, the armpit inductive sensor 241, the armpit inductive sensor 242, the elbow inductive sensor 251, the elbow inductive sensor 252, the waist inductive sensor 261, the waist inductive sensor 262, a waist inductive sensor 663, the abdominal inductive sensor 270, the right hip sensor 411, the left hip sensor 412, the right front hip sensor 413, the left front hip sensor 414, the crotch inductive sensor 420, the right knee inductive sensor 431, and the left knee inductive sensor 432.

In some embodiments, the plurality of inductive sensors disposed on the wearable body 110 may be omitted to simplify the structure of the wearable body 110. For example, since a sensitivity of a variation in an inductance value caused by the movements of the waist and the abdomen is relatively small, the waist inductive sensor 261, the waist inductive sensor 262, the waist inductive sensor 663, and the abdominal inductive sensor 270 may be omitted. As another example, the waist inductive sensor may be combined with the adjacent right hip sensor 411 and the left hip sensor 412.

In some embodiments, in order to prevent interference between the adjacent sensors, a distance between every two inductive sensors of the plurality of inductive sensors disposed at different positions or joints may be greater than 3 cm. In some embodiments, in order to prevent interference between the adjacent sensors, the distance between every two inductive sensors of the plurality of inductive sensors disposed at different positions or joints may be greater than 4 cm.

FIG. 8 is a block diagram illustrating an exemplary wearable apparatus according to some embodiments of the present disclosure. As shown in FIG. 8, the wearable apparatus 100 may include the main control circuit board 210 (e.g., the main control circuit board 210 shown in FIG. 2 or FIG. 6) and a plurality of sensors 830. The main control circuit board may further include the readout system 120 (e.g., the distributed readout system 120) and the processing circuit 130. The plurality of sensors 830 may be the plurality of inductive sensors distributed on the wearable body 110 shown in FIGs. 2-7, or may be other types of sensors (e.g., a capacitive sensor or a resistive sensor).

The distributed readout system 120 may be configured to read data from at least a portion of the plurality of sensors 830. In some embodiments, the distributed readout system 120 may include a plurality of readout units. Each of the plurality of readout units may be connected with at least one of the plurality of sensors 830 through a conductive wire. In some embodiments, the distributed readout system 120 may include a plurality of subsystems. Each of the plurality of subsystems may be connected with at least one of the plurality of sensors 830. For example, each of the plurality of subsystems may be connected with a series of related sensors 830 (e.g., a certain readout unit may be connected with all inductive sensors crossing a waist contour. For example, the certain readout unit may be connected with the waist inductive sensor 261, the waist inductive sensor 262, and the abdominal inductive sensor 270. Each of the plurality of subsystems may be connected with the processing circuit 130 of the main control circuit board via a conductive wire, Bluetooth, WIFI, etc. In some embodiments, each of the plurality of readout units may obtain an electrical signal from the connected sensor 830 and transmit the electrical signal to the processing circuit 130. In some embodiments, the distributed readout system 120 may include at least two readout units. It is understood that a series of related inductive sensors may be all inductive sensors (e.g., all sensors enclosing a shoulder joint) enclosing the same joint/part at the same time, or may be all sensors (e.g., all sensors near the waist) of which distances are less than a preset threshold.

The processing circuit 130 may be configured to generate a parameter reflecting a joint movement based on the data. For example, the processing circuit 130 may receive variable inductance data sent by the distributed readout system 120, and convert the variable inductance data into the parameter reflecting the joint movement. Exemplary parameters reflecting the joint movement may include a joint bending angle, a joint movement direction, a joint displacement distance, a joint flexion and extension situation, etc. The joint bending angle in the present disclosure may be an angle formed by skin surfaces on both sides of the joint during a deformation process of the joint. In some embodiments, the electrical signals generated by the plurality of sensors 830 may have a corresponding relationship with joint movement information. The processing circuit 130 may determine what kind of action occurs at the corresponding joint based on the variable electrical signals generated by the plurality of sensors 830. In some embodiments, the processing circuit 130 may include a wireless transmission unit, a power module, and a data processing module.

In some embodiments, the processing circuit 130 may be connected with a terminal device. For example, the processing circuit 130 may be connected with the terminal device via a network, a data line (a data interface), a wireless transmission unit, etc. The processing circuit 130 may generate the parameter reflecting the joint movement by processing the electrical signals (e.g., an inductance value, a circuit impedance, etc.) generated by the plurality of sensor 830, and send the parameter to the terminal device. The terminal device may include a mobile phone, a computer, or other smart devices.

FIG. 9 is a schematic structural diagram illustrating an exemplary readout unit according to some embodiments of the present disclosure.

As shown in FIG. 9, the readout system 120 may include a readout chip (e.g., an FDC2214 chip) and a capacitor $C_0$. The readout chip may send an excitation signal to an LC resonant system and read a resonant frequency of the LC resonant system, to calculate the inductance value of each of the plurality of inductive sensors. The capacitor $C_0$ refers to a large constant capacitor having a capacitance value of $C_0$ connected in parallel with the plurality of inductive sensors. In this case, the resonant frequency of the LC resonant system may be shown in the following formula (1):

$$f_0 = \frac{1}{2\pi}\sqrt{\frac{2}{L_s(C_s+C_0)}} \quad (1)$$

wherein Ls denotes an inductance of each of the plurality of inductive sensors, and Cs denotes a capacitance of each of the plurality of inductive sensors.

It should be noted that in a resonance measurement method, in order to improve the stability and accuracy of results, it is necessary to consider a parallel capacitance value and a Q value (which depends on the readout chip) of the LC resonant system. The parallel capacitance value is inversely proportional to the Q value of the LC resonant system. The greater the parallel capacitance value, the less the LC resonant system is subjected to an external capacitance interference (e.g., a parasitic capacitance generated by a human hand touching one of the plurality of inductive sensors), and the higher the reliability of the inductive sensor. However, the smaller the Q value, the lower the accuracy of the measured resonant frequency. In some embodiments, the parallel capacitance value may be within a range of 100 pF-10 nF, and the Q value corresponding to the LC resonant system may be within a range of 2-100.

The wearable apparatus 100 described in some embodiments of the present disclosure can reduce the influence of the parasitic capacitance on the resonant frequency of each of the plurality of sensors 830 by connecting the large constant capacitor in parallel to each of the plurality of sensors 830 and then measuring the resonant frequency of the LC resonant system by the resonance measurement method, thereby improving the anti-interference ability. Meanwhile, by considering the parallel capacitance value and the Q value of the LC resonant system, both of the anti-interference ability of the wearable apparatus and the measurement accuracy of the resonant frequency can be considered.

FIG. 10 is a schematic structural diagram illustrating an exemplary inductive sensor according to some embodiments of the present disclosure. As shown in FIG. 10, an inductive sensor 1000 may include an inductive structure enclosed by a conductive wire 1010, a first readout lead 1021, and a second readout lead 1022. In some embodiments, the inductive sensor 1000 may be fixed to a wearable body 1030 by stitching, weaving, pressing, pasting, snapping, etc.

In some embodiments, a material of the conductive wire 1010 may cause an elastic deformation or a plastic deformation under a small stress to ensure that the conductive wire 1010 may generate a deformation accordingly with a movement of a joint. Exemplary materials of the conductive wire 1010 may include silver, copper, aluminum, an alloy material, a composite materials, or the like, or any combination thereof.

In some embodiments, the conductive wire 1010 may include an elastic and stretchable conductive yarn. The conductive yarn may be fixed by weaving. For example, the conductive yarn may be directly woven at a joint position of the wearable body. In some embodiments, the conductive yarn may be woven on an inner side and/or an outer side of the joint position of the wearable body. By fixing the elastic and stretchable conductive yarn by weaving, the foreign body sensation when a user wears the wearable body can be reduced, the user experience can be improved, and the firmness of fixation can be improved to prevent the sensor from falling off. It is understood that the inductive sensor 1000 may also be fixed to the wearable body by other forms such as suturing, pressing, pasting, and snapping, which is not limited in the present disclosure.

In some embodiments, in order to avoid an external short circuit of the conductive wire 1010 and protect the conductive wire 1010, a surface of the conductive wire 1010 may be wrapped with at least one layer of insulating material. Exemplary insulating materials may include polyvinyl chloride, cross-linked polyethylene, ethylene propylene rubber, silicone rubber, fluoroplastics, insulating cloth, insulating colloid, etc. The insulating material may wrap the conductive wire 1010 by gluing, coating, or the like, or other ways.

In some embodiments, the conductive wire 1010 may form a spiral inductive pattern enclosing the joint position. In some embodiments, the inductive pattern may be a planar spiral inductive pattern. For example, a shape of the inductive pattern may include but is not limited to a square, a rectangle, a circle, a polygon, a semicircle, a circle, an ellipse, or other planar shapes. A spiral direction of the inductive pattern may be clockwise or counterclockwise. In some embodiments, an inductance of the inductive structure may be calculated by the following formula (2):

$$L = \frac{N\varnothing}{l} = \frac{\mu N^2 S}{l} \quad (2),$$

wherein L denotes the inductance, ø denotes a magnetic flux, $\mu$ denotes a magnetic permeability, N denotes a count of turns of a coil (a number of turns), S denotes an effective area through which magnetic lines of force in the inductance pattern pass, and *l* denotes a length of the coil. It can be seen from the above formula that when the joint extends, the inductive pattern may be in a fully unfolded state (i.e., the inductive pattern may be laid flat on the wearable body). In this case, the effective area S may be maximum, and the corresponding inductance L may also be maximum. When the joint is bent, the inductive pattern may be in a bent state (i.e., the inductive pattern generates a partial deformation). In this case, the effective area S may decrease, and the corresponding inductance L may also decrease. In some embodiments, when the inductive pattern is in the bent state, the inductive pattern may partially overlap, wrinkle, bulge, etc., so a variation in the effective area may be nonlinear. In addition, if the wearing body does not fit, the variation in the effective area may also be nonlinear. Therefore, when the joint of the user is bent to deform, the inductance may vary nonlinearly with the variation in the effective area S.

In some embodiments, the spiral inductive pattern may include more than one turn of the conductive wire. For example, spiral inductive pattern may include 2 turns, 5 turns, 10 turns, 50 turns, etc., of the conductive wire. Preferably, the spiral inductive pattern may include more than two turns of the conductive wire.

In some embodiments, an angle between a long axis direction of the spiral inductive pattern and a bending axis of the joint may be within a range of 90 degrees ± 20 degrees, and an angle between a short axis direction of the spiral inductive pattern and the bending axis of the joint may be within a range of ± 20 degrees. For example, the angle between the long axis direction of the spiral inductive pattern and the bending axis of the joint may be within a range of 80 degrees-100 degrees, and the angle between the short axis direction of the spiral inductive pattern and the bending axis of the joint may be within a range of ± 10 degrees. As another example, the angle between the long axis direction of the spiral inductive pattern and the bending axis of the joint may be within a range of 85 degrees-95 degrees, and the angle between the short axis direction of the spiral inductive pattern and the bending axis of the joint may be within a range of ± 5 degrees. As another example, the long axis direction of the spiral inductive pattern may be perpendicular to the bending axis of the joint, and the short axis direction of the spiral inductive pattern may be parallel to the bending axis of the joint. In some embodiments, the bending axis of the joint refers to a straight line where all fixed points of the joint are located during the deformation process.

In some embodiments, in order to optimize the sensitivity of the inductive sensor 1000, the shape of the spiral inductive pattern may be designed symmetrically according to the long axis or the short axis. For example, the spiral inductive pattern may be a rectangle, a circle, an ellipse, etc. with the long axis and the short axis as an axis of symmetry.

In some embodiments, the greater the thickness of the conductive yarn in a direction perpendicular to the surface of the inductive pattern, the stronger the foreign body sensation generated when the user wears the wearable apparatus; and the greater thickness increases a stress required for the deformation of the conductive yarn, and the conductive yarn may be difficult to generate the corresponding deformation when the joint makes a small movement. In order to improve the comfort of the user wearing the wearable apparatus and improve the sensitivity of the inductive sensor 1000, the thickness of the conductive yarn in the direction perpendicular to the surface of the inductive pattern may not be greater than 3 mm. For example, the thickness of the conductive yarn in the direction perpendicular to the surface of the inductive pattern may be 2.5 mm, 2 mm, 1.5 mm, 1 mm, etc. Preferably, the thickness of the conductive yarn in the direction perpendicular to the surface of the inductive pattern may be 2 mm.

In some embodiments, an inner circle and an outer circle of the spiral inductive pattern may be provided with a readout lead, respectively, for connecting the inductive structure to a readout unit. In some embodiments, as shown in FIG. 10, the inner circle of the spiral inductive pattern may be a portion of the conductive wire connected with the second readout lead 1022. The outer circle of the spiral inductive pattern may be a portion of the conductive wire connected with the first readout lead 1021.

The readout lead may be a portion (e.g., an end portion of the conductive wire) of the conductive wire. As shown in FIG. 2, the readout lead may include the first readout lead 1021 and the second readout lead 1022. In some embodiments, the readout lead may be connected with the readout unit. For example, the readout lead may be connected with the readout unit through a data interface.

In some embodiments, in order to improve the Q value of an inductor and then improve the measurement accuracy, a resistance of the inductive structure may be less than 100 Ω. For example, the resistance of the inductive structure may be less than 80 Ω. As another example, the resistance of the inductive structure may be less than 50 Ω. As another example, the resistance of the inductive structure may be less than 30 Ω. As another example, the resistance of the inductive structure may be less than 10 Ω.

FIG. 11 is a schematic diagram illustrating a top view of an exemplary inductive sensor according to some embodiments of the present disclosure.

An inductive sensor 1100 may include a spiral inductive coil 1121 and a substrate 1122. In some embodiments, under a deformation of a joint of a user, the spiral inductive coil 1121 and the substrate 1122 may also generate a deformation, and generate an electrical signal based on the deformation. Exemplary electrical signals may include an inductance, a circuit impedance, a phase, a resonant frequency, or the like, or any combination thereof. Merely by way of example, the inductive sensor 1100 may measure a shape variation of the inductive sensor 1100 (or the spiral inductive coil 1121) with a movement of the human joint using a variation in an inductance value of the spiral inductive coil 1121, to determine the movement of the joint corresponding to the inductive sensor 1100. For example, when the user wears a wearable apparatus, at least a portion of the inductive sensor 1100 may be disposed at the joint of the user to collect a movement signal of the user to determine the movement. Specifically, when the joint of the user wearing the wearable apparatus performs a specific movement, a shape of the inductive sensor 1100 may change to cause a variation in the inductance value of the spiral inductive coil 1121 of the inductive sensor 1100 and obtain joint movement information (e.g., a bending angle of the joint, a flexion and extension situation of the joint) of the user through the variation in the inductance value, thereby capturing the movement of the joint of the user.

The substrate 1122 may be configured to carry the spiral inductive coil 1121. In some embodiments, a material of the substrate 1122 may include but is not limited to flexible organic film materials such as PI, PET, silicone, and rubber. In some embodiments, the substrate 1122 may use a flexible printed circuit (FPC). In some embodiments, the substrate 1122 may also be made directly of textile fabrics to enhance the comfort of the user when wearing the wearable apparatus. A thickness of the substrate 1122 needs to be considered between user comfort and practicality. If the substrate 1122 is too thick, the user may not be comfortable enough. If the substrate 1122 is too thin, the substrate may wrinkle, affecting the reading accuracy of the inductance value. In some embodiments, the thickness of the substrate 1122 may be within a range of 1 μm-500 μm.

In some embodiments, the spiral inductive coil 1121 may include a single layer coil. In this case, a count of coils of the conductive wire of the spiral inductive coil 1121 may be greater than or equal to 2. In some embodiments, in order to increase a total inductance value of the inductive sensor 1100, the spiral inductor 1121 may include a plurality layers of coils with the same current direction. The plurality layers of coils with the same current direction may be disposed in layers in a direction perpendicular to the substrate 1122. Optionally, projections of the plurality layers of coils in the direction perpendicular to the substrate 1122 may partially overlap or completely overlap. Further, by increasing an overlap degree of the projections of the plurality layers of coils in the direction perpendicular to the substrate 1122, the inductance value of the inductive sensor 1100 may be increased, thereby improving the reliability of the inductive sensor 1100. In some embodiments, a shape (i.e., an overall shape of a pattern (or an inductive pattern) formed by winding the conductive wire) of the spiral inductive coil 1121 may be a regular geometric shape such as a rectangle, a circle, an ellipse, or other irregular shapes. Preferably, the shape of the spiral inductive coil 1121 may be an axisymmetric geometric shape.

In some embodiments, a material of the inductive wire for winding the spiral inductive coil 1121 may be metal/alloy, or a conductive material such as silver paste, carbon paste, ITO, liquid metal, etc. A spiral winding direction of the conductive wire may be clockwise or counterclockwise.

The wearable apparatus described in some embodiments of the present disclosure may sense the hand movement of the user by using the inductive sensor 1100, and may not be easily interfered by external factors such as temperature, humidity, pressure, and sweat. In addition, the inductive sensor 1100 may be obtained by disposing the spiral inductive coil 1121 on the substrate 1122, which is easy to prepare, low in cost, and suitable for industrial production. Furthermore, the inductance (a higher inductance may be achieved based on a smaller size) of the inductive sensor 1100 may be increased by increasing the count of coils of the spiral inductive coil 1121, thereby improving the sensitivity of the sensor, meeting the application requirements of the small size of the sensor, and simplifying the design of the subsequent readout system.

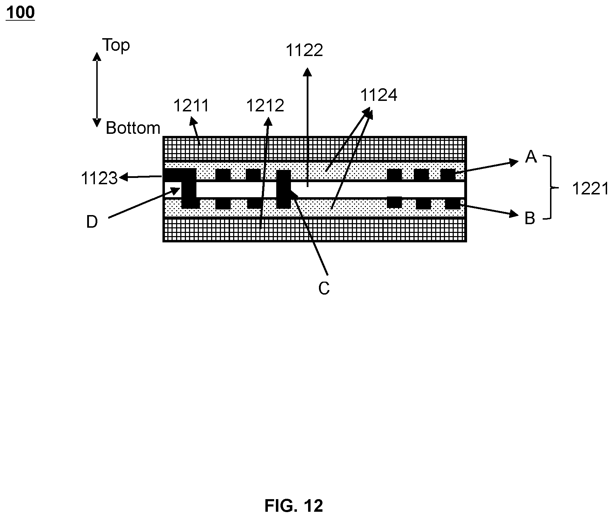

FIG. 12 is a schematic diagram illustrating a cross section of an exemplary wearable apparatus according to some embodiments of the present disclosure.

As shown in FIG. 12, in some embodiments, the wearable apparatus may include a fabric layer 1211 and a fabric layer 1212. Each of a plurality of inductive sensors of the wearable apparatus may include a spiral inductive coil 1221, a substrate 1122, a signal lead-out terminal 1123, and a protective layer 1124. In some embodiments, in order to increase a total inductance of the plurality of inductive sensors and then increase sensitivities of the plurality of inductive sensors, the spiral inductive coil 1221 may include a plurality layers of coils. For example, the spiral inductive coil 1221 may include at least a first layer coil A and a second layer coil B. As another example, the spiral inductive coil 1221 may include three layers of inductive coils, four layers of inductive coils, or more layers of inductive coils.

Taking the spiral inductive coil 1221 including the first layer coil A and the second layer coil B as an example, a current direction in the first layer coil A and a current direction in the second layer coil B may be the same. Optionally, projections of areas enclosed by the plurality of coils in a direction perpendicular to the substrate 1122 may partially overlap or completely overlap. In some embodiments, the projections of the areas enclosed by the plurality of coils in the direction perpendicular to the substrate 1122 may not overlap. If an inductive current in the first layer coil A and an inductive current in the second layer coil B are in opposite directions, an inductance of the first layer coil A and an inductance of the second layer coil B may cancel each other, thereby reducing the total inductance value of the plurality of inductive sensors. If the inductive current in the first layer coil A and the inductive current in the second layer coil B are in the same direction, it is ensured that the total inductance L of the plurality of inductive sensors is a sum of an inductance L1 of the first layer coil A, an inductance L2 of the second layer coil B, and a mutual inductance M of the first layer coil A and the second layer coil B, as shown in the following formula (3):

$$L = L1 + L2 + 2M \quad (3).$$

In some embodiments, in order to simplify the structures of the plurality of inductive sensors and reduce the count of lead-out terminals of the plurality of inductive sensors to save cost, the first layer coil A and the second layer coil B may be disposed on both sides of the substrate, respectively. For example, the substrate 1122 may be provided with a through hole C, and the first layer coil A may be connected with the second layer coil B through the through hole C. That is, the first layer coil A and the second layer coil B may be respectively formed by a same wire passing through the through hole C. For example, the first layer coil A on an upper side of the substrate 1122 may be wound from an outer side to an inner side of the substrate 1122, and then led to a lower side of the substrate 1122 through the through hole C. The second layer coil B may be wound from the inner side to the outer side of the substrate 1122, so that the current direction in the first layer coil A and the current direction in the second layer coil B may be finally the same (e.g., clockwise or counterclockwise).

In some embodiments, the first layer coil A and the second layer coil B may also be wound by a wire, respectively. The current directions in two wires for winding the first layer coil A and the second layer coil B may be in the same direction (e.g., clockwise or counterclockwise). For example, the through hole C may not be provided in the substrate 1122. The substrate 1122 may be provided with four lead terminals. Each layer coil may correspond to two of the four lead terminals. For the first layer coil A on the upper side of the substrate 1122, the first layer coil A may be wound from the outer side to the inner side from one lead terminal corresponding to the first layer coil A, and pass through each coil from the inner side to the other lead terminal corresponding to the first layer coil A. For the second layer coil B on the lower side of the substrate 1122, the second layer coil B may also be wound from the outer side to the inner side from one lead terminal corresponding to the second layer coil B, and pass through each coil to the other lead terminal corresponding to the second layer coil B. Alternatively, the second layer coil B on the lower side of the substrate 1122 may also be led from one lead terminal corresponding to the second layer coil B to the inner side of the second layer coil B, and then wound from the inner side to the outer side to the other lead terminal corresponding to the second layer coil B.

In some embodiments, signals at both ends of the same wire for winding the first layer coil A and the second layer coil B may be respectively led out from both sides of the substrate 1122. In some embodiments, in order to flexibly set positions of the lead-out terminals of the plurality of inductive sensors, the substrate 1122 may be provided with a through hole D. The signals at both ends of the inductive wire (the same wire for winding the first layer coil A and the second layer coil B) may be led out from the same side of the substrate 1122 through the through hole D.

The protective layer 1124 may be configured to package a spiral inductive coil. The protective layer 1124 may be made of a wear-resistant, corrosion-resistant, and waterproof material. The protective layer 1124 may provide a good electrical environment for the spiral inductive coil.

The plurality of inductive sensors described in some embodiments of the present disclosure can increase the total inductance of the plurality of inductive sensors by setting two or more layers of inductances on the upper and lower sides of the substrate 1122, which is beneficial to the stability and accuracy of the subsequent readout system while improving the sensitivities of the plurality of inductive sensors, and is easy to implement the process.

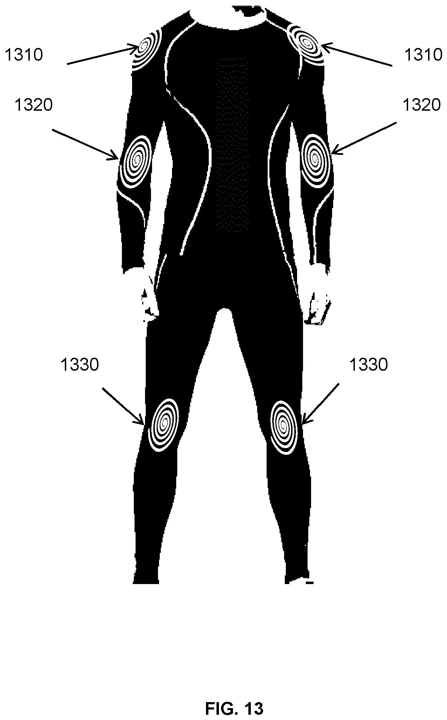

FIG. 13 is a schematic diagram illustrating a user wearing an exemplary wearable apparatus according to some embodiments of the present disclosure. As shown in FIG. 13, a plurality of inductive sensors (referred to as plurality of sensors) of the wearable apparatus may be attached to a wearable body at positions corresponding to a shoulder joint 1310, an elbow joint 1320, and a knee joint 1330, respectively.

In some embodiments, the plurality of sensors may be attached to the wearable body 110 in a detachable manner. For example, the plurality of sensors may also be attached to the wearable body 110 in other forms such as gluing, bundling, snapping, touch fasteners, etc. In some embodiments, a size or shape of an inductive structure of each of the plurality of sensors may be adjustable. For example, the size of the inductive structure may be adaptively adjusted to cover all movable positions of a joint position according to an arm width, a shoulder width, and a knee width of the joint position of the user. In some embodiments, the size of the inductive structure may be slightly smaller than a size of the joint position to avoid the error effect on a variable inductance caused by a deformation of a redundant part of the inductive structure such as extrusion, twisting, overlapping, etc.

In some embodiments, at the same joint, each of the plurality of sensors may include two inductive structures disposed at inner and outer sides of the joint respectively. For example, for the elbow joint 1320, the sensor may include an outer joint inductive structure and an inner joint inductive structure which are respectively attached to an outer side and an inner side of the elbow joint corresponding to the wearable body. In some embodiments, a size of the inductive structure disposed at the outer side of the joint may be slightly larger than a size of the inductive structure disposed at the inner side of the same joint to adapt to the deformation of a joint movement of the user. In some embodiments, when at least two inductive structures measure an inductance value of the same joint, a final measurement result may be a calculated value (e.g., a mean, a weighted average, etc.) based on measurement results measured by the two inductive structures, respectively. For example, when the knee is bent once, the inductive structure disposed at the outer side of the knee may measure an outer side inductance value, and the inductive structure disposed at the inner side of the knee may measure an inner side inductance value, and then the final measurement result may be the mean or the weighted average of the outer side inductance value and the inner side inductance value. In some embodiments, in order to improve the accuracy of measurement, a plurality of inductive structures may be disposed at the same joint. When at least two inductive structures measure the inductance value of the same joint, a plurality of output inductance values may be processed by the readout system 120 and the processing circuit 130, respectively to obtain a plurality of bending degrees of the joint. The mean or the weighted average of the plurality of bending degrees of the joint may be obtained, and the mean or the weighted average may be used as the final measurement result. In some embodiments, in order to improve the accuracy of measurement, the size, the shape, a fitting position, etc. of the inductive structure may be changed to calibrate or test the sensor. For example, an inductive structure with a rectangular spiral inductive pattern and an inductive structure with an elliptical spiral inductive pattern may be used for measurement of the same joint, and measurement results may be calibrated at different bending angles of the joint.

It should be noted that, for the convenience of explanation, FIG. 13 is an exemplary structural diagram when the wearable body 110 is sportswear or sports pants. In addition, the wearable body 110 may also be other forms of components such as a wrist guard, an elbow guard, a knee pad, a shoulder guard, etc., of which functions and principles may similar to those of the sportswear and the sports pants. On the premise of understanding the solution of the present disclosure, those having skills in the art may apply the solution to any suitable scenario.

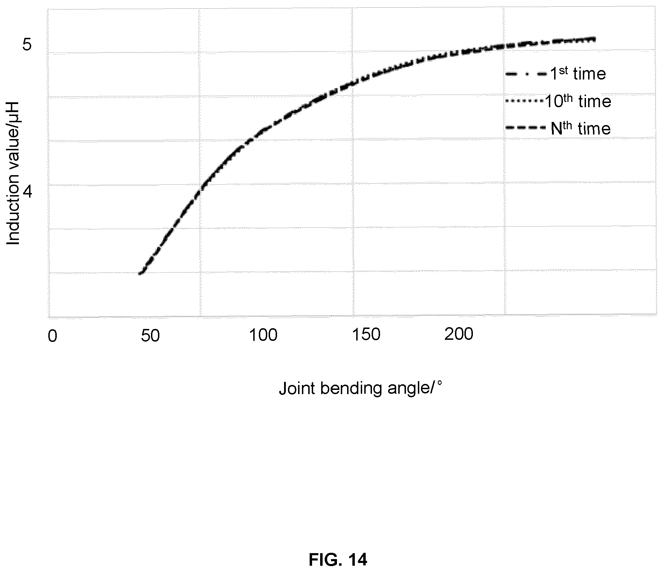

FIG. 14 is a schematic diagram illustrating a curve showing a variation in an inductance value of an exemplary inductive structure having a spiral inductive pattern with a bending angle of the inductive structure in a non-wearing state according to some embodiments of the present disclosure. The bending angle of the inductive structure described in FIG. 14 refers to an angle formed by two surfaces of the inductive structure during a folding process of the inductive structure in a short axis direction (e.g., the short axis direction in FIG. 10) in a non-wearing state. As shown in FIG. 14, when a 1 V voltage and a 1 kHz alternating current are applied to the inductive structure, the inductance value of the inductive structure having the spiral inductive pattern may increase nonlinearly with an increase of the bending angle of the inductive structure. Further, when the bending angle of the inductive structure is 30°, the inductance value may be 3.99 $\mu$H; when the bending angle of the inductive structure is 60°, the inductance value may be 4.52 $\mu$H; when the bending angle of the inductive structure is 90°, the inductance value is may be 4.79 $\mu$H; when the bending angle of the inductive structure is 120°, the inductance value may be 4.94 $\mu$H; when the bending angle of the inductive structure is 150°, the inductance value may be 5.02 $\mu$H; and when the bending angle of the inductive structure is 180°, the inductance value may be 5.05 $\mu$H. In some embodiments, in order to test the reusability of the inductive structure, the same inductive structure may be repeatedly measured multiple times (e.g., N times). As shown in FIG. 14, results of multiple measurements show that in different measurement processes, the same bending angle of the inductive structure may correspond to the same inductance value, and a relationship curve of the inductance value-bending angle after N times of bending may be always consistent. Therefore, the inductance value of the inductive structure does not drift with the repeated use of the material or factors such as temperature, and has good reusability.

In some embodiments, the inductance value of the inductive structure having the spiral inductive pattern may also has a one-to-one correspondence relationship with the bending angle of the joint in a wearing state. For example, a sensor for an elbow may be used for a movement of the elbow joint, and an AC current of a 1 V voltage and a 1 kHz may be applied to the inductive structure. When the bending angle of the joint is 180° (i.e., the hand is stretched flat), the inductance value may be 3.91 $\mu$H; when the bending angle of the joint is 90°, the inductance value may be 2.91 $\mu$H; and when the bending angle of the joint is 50°-60°, the inductance value may be 2.03 $\mu$H.

In some embodiments, since wearing may cause a deformation of the wearable apparatus and the inductive pattern, for the same inductive structure, in the wearing and non-wearing states, the same bending angle (e.g., the bending angle of the inductive structure in the non-wearing state may be equal to the bending angle of the joint in the wearing state) may correspond to different inductance values. Therefore, in order to meet the accurate measurement of a movement state, before the user performs measurement in the movement state, the inductive structure may be calibrated to obtain a variation curve of the joint bending degree and the corresponding inductance value of the user. For example, when the user make different joint bending angles, the corresponding inductance values are measured to perform a curve fitting, and the variation curve of the joint bending degree and the inductance value of the user is obtained. In some embodiments, due to different body shapes, different users may have different inductance values when wearing the same inductive structure at the same joint bending angle. Therefore, in order to meet the accurate measurement of the movement state of different people, before each user performs measurement in the movement state, the inductive structure may be calibrated to obtain the variation curve of the joint bending degree and the inductance value corresponding to each user. For example, when each user make different joint bending angles, the corresponding inductance values are measured to perform a curve fitting, and the variation curve of the joint bending degree and the inductance value of the each user is obtained. In the subsequent measurement process of the movement state of the user, accurate measurement of the movement state may be achieved based on the variation curve of the joint bending degree and the inductance value.

In order to meet the different measurement of the movement state at different joint positions, in some embodiments, the corresponding relationship (i.e., a nonlinear change relationship) between the bending angle of the inductive structure at different joint positions and the corresponding inductance value may be different. For example, in order to meet the accurate measurement of the movement state of the knee joint, compared with the elbow joint, the inductive structure disposed at the knee joint position and the inductive structure disposed at the elbow joint position may have different counts of windings, different effective areas, and/or different coil lengths, such that the inductive structure disposed at the knee joint position may generate a greater inductance value than the inductive structure disposed at the elbow joint position at the same joint bending angle.

In some embodiments, in order to facilitate processing and reduce the processing burden of the processing circuit or the processing device, the inductive structures disposed at different joint positions may generate equal or substantially equal inductance values at the same joint bending angle. For example, by designing the inductive structure disposed at the knee joint position to have a larger effective area and fewer counts of turns, and designing the inductive structure disposed at the elbow joint position to have a smaller effective area and more counts of turns, the bending angles of the inductive structure disposed at the knee joint, the bending angles of the inductive structure disposed at the elbow joint positions, the corresponding inductance values of the inductive structure disposed at the knee joint, and the corresponding inductance values of the inductive structure disposed at the knee joint may present the same or substantially the same nonlinear change relationship, which is convenient for subsequent processing of the processing circuit or the processing device.

The beneficial effects brought about by the wearable apparatus described in some embodiments of the present disclosure include but are not limited to the following descriptions. (1) With the wearable apparatus described in some embodiments of the present disclosure, the joint movements of the user can be converted into the variable electrical signals, and then the parameters reflecting the joint movements can be generated, thereby accurately capturing the movements of the user. (2) The plurality of inductive sensors can be obtained by winding the spiral inductive coils on the substrate, making the wearable apparatus easy to prepare and low in cost. (3) By using the flexible material, the thin-film flexible inductive sensor can be obtained, which is comfortable to wear. (4) The plurality of inductive sensors only respond to the shape variation (e.g., the detection result can only be related to the inductance values), such that the wearable apparatus has a strong anti-interference ability to external factors such as temperature, humidity, pressure, sweat, etc., thereby improving the accuracy and reliability of movement capture and the reusability of the wearable apparatus. (5) By controlling the distance between the plurality of inductive sensors, mutual influence between the plurality of inductive sensors can be prevented. (6) By controlling the positions of the inductive structures of the plurality of inductive sensors, the accuracy of capture and restoration of the joint movements can be improved.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and "some embodiments" mean that a particular feature, structure, or feature described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or features may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the spirit and scope of the disclosed embodiments. For example, although the implementation of various parts described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

In some embodiments, numbers describing the number of ingredients and attributes are used. It should be understood that such numbers used for the description of the embodiments use the modifier "about", "approximately", or "substantially" in some examples. Unless otherwise stated, "about", "approximately", or "substantially" indicates that the number is allowed to vary by $\pm 20\%$. Correspondingly, in some embodiments, the numerical parameters used in the description and claims are approximate values, and the approximate values may be changed according to the required features of individual embodiments. In some embodiments, the numerical parameters should consider the prescribed effective digits and adopt the method of general digit retention. Although the numerical ranges and parameters used to confirm the breadth of the range in some embodiments of the present disclosure are approximate values, in specific embodiments, settings of such numerical values are as accurate as possible within a feasible range.

For each patent, patent application, patent application publication, or other materials cited in the present disclosure, such as articles, books, specifications, publications, documents, or the like, the entire contents of which are hereby incorporated into the present disclosure as a reference. The application history documents that are inconsistent or conflict with the content of the present disclosure are excluded, and the documents that restrict the broadest scope of the claims of the present disclosure (currently or later attached to the present disclosure) are also excluded. It should be noted that if there is any inconsistency or conflict between the description, definition, and/or use of terms in the auxiliary materials of the present disclosure and the content of the present disclosure, the description, definition, and/or use of terms in the present disclosure is subject to the present disclosure.

Finally, it should be understood that the embodiments described in the present disclosure are only used to illustrate the principles of the embodiments of the present disclosure. Other variations may also fall within the scope of the present disclosure. Therefore, as an example and not a limitation, alternative configurations of the embodiments of the present disclosure may be regarded as consistent with the teaching of the present disclosure. Accordingly, the embodiments of the present disclosure are not limited to the embodiments introduced and described in the present disclosure explicitly.

## Claims

1. A wearable apparatus, comprising:
   a wearable body, wherein a plurality of inductive sensors are distributed on the wearable body, the plurality of inductive sensors include a plurality of inductive sensors crossing a joint contour, each of the plurality of inductive sensors includes an inductive structure that is enclosed by a conductive wire, and each of the plurality of inductive sensors is attached to a position of the wearable body corresponding to a joint position and generates variable inductances with deformations of the joint position.

2. The wearable apparatus of claim 1, wherein the inductive structure includes a long axis direction and a short axis direction, and the joint contour passes through a 1/3 middle region of the inductive structure in the long axis direction.

3. The wearable apparatus of claim 1, wherein the plurality of inductive sensors include a plurality of inductive sensors crossing an arm root contour, each of the plurality of inductive sensors crossing the arm root contour includes a first inductive structure enclosed by a first conductive wire, and the first inductive structure is configured to generate variable inductances with movements of a shoulder joint of a user.

4. The wearable apparatus of claim 3, wherein the plurality of inductive sensors crossing the arm root contour include:

an inductive sensor disposed in front of a shoulder,

an inductive sensor disposed at the rear of a shoulder, and

an inductive sensor disposed under an armpit.

5. The wearable apparatus of claim 4, wherein a distance between every two inductive sensors of the plurality of inductive sensors crossing the arm root contour is greater than 3 cm.

6. The wearable apparatus of claim 4, wherein the wearable body further includes a capacitive sensor or a resistive sensor distributed near the shoulder joint, the capacitive sensor is configured to generate variable capacitances with movements of a shoulder joint of a user, and the resistive sensor is configured to generate variable resistances with the movements of the shoulder joint of the user.

7. The wearable apparatus of claim 3, wherein the first inductive structure includes a long axis direction and a short axis direction, and the arm root contour passes through a 1/3 middle region of the first inductor structure in the long axis direction.

8. The wearable apparatus of claim 1, wherein the plurality of inductive sensors include a plurality of inductive sensors crossing an elbow contour, each of the plurality of inductive sensors crossing the elbow contour includes a second inductive structure enclosed by a second conductive wire, and the plurality of inductive sensors crossing the elbow contour are configured to generate variable inductances with movements of an elbow joint of a user.

9. The wearable apparatus of claim 8, wherein the second inductive structure includes a long axis direction and a short axis direction, and the elbow contour passes through a 1/3 middle region of the second inductive structure in the long axis direction.

10. The wearable apparatus of claim 8, wherein a distance between every two inductive sensors of the plurality of inductive sensors crossing the elbow contour is greater than 3 cm.

11. The wearable apparatus of claim 1, wherein the plurality of inductive sensors include a plurality of inductive sensors crossing a waist contour, each of the plurality of inductive sensors crossing the waist contour includes a third inductive structure enclosed by a third conductive wire, and the plurality of inductive sensors crossing the waist contour are symmetrically distributed with respect to a central sagittal plane of a user in a wearing state and the plurality of inductive sensors are configured to generate variable inductances with movements of waist of the user.

12. The wearable apparatus of claim 11, wherein the third inductive structure includes a long axis direction and a short axis direction, and the waist contour passes through a 1/3 middle region of the third inductive structure in the long axis direction.

13. The wearable apparatus of claim 11, wherein a distance between every two inductive sensors of the plurality of inductive sensors crossing the waist contour is greater than 3 cm.

14. The wearable apparatus of claim 1, wherein the plurality of inductive sensors include a plurality of inductive sensors crossing a knee contour, and the plurality of inductive sensors crossing the knee contour are disposed on an inner side or an outer side of a knee joint of a user in a wearing state and the plurality of inductive sensors are configured to generate variable inductances with movements of the knee joint of the user.

15. The wearable apparatus of claim 14, wherein each of the plurality of inductive sensors crossing the knee contour includes a fourth inductive structure enclosed by a fourth conductive wire, the fourth inductive structure includes a long axis direction and a short axis direction, and the knee contour passes through a 1/3 middle region of the fourth inductive structure in the long axis direction.

16. The wearable apparatus of claim 14, wherein a distance between every two inductive sensors of the plurality of inductive sensors crossing the knee contour is greater than 3 cm.

17. The wearable apparatus of claim 1, wherein the wearable body is further provided with a plurality of inductive sensors disposed at a hip bone position, and the plurality of inductive sensors disposed at the hip bone position are configured to generate variable inductances with movements of a hip joint of a user.

18. The wearable apparatus of claim 17, wherein the plurality of inductive sensors disposed at the hip bone position include a left hip sensor and a right hip sensor, and in a wearing state, a connection line between a highest point of a left hip bone and a highest point of a right hip bone of the user passes through the left hip sensor and the right hip sensor, respectively.

19. The wearable apparatus of claim 17, wherein a distance between every two inductive sensors of the plurality of inductive sensors disposed at the hip bone position is greater than 3 cm.

20. The wearable apparatus of claim 17, wherein the wearable body further includes a crotch sensor disposed under a crotch, a left front hip sensor disposed at a left front position of the hip bone, and a right front hip sensor disposed at a right front position of the hip bone.

21. The wearable apparatus of claim 20, wherein intersection points of a connection line between a highest point of a left hip bone and a highest point of a right hip bone of the user and an outer edge of the wearable body are a first intersection point and a second intersection point, respectively, wherein

   a connection line between the first intersection point and a center point of the crotch passes through a 1/3 middle region the left front hip sensor in the long axis direction, and
   a connection line between the second intersection point and the center point of the crotch passes through a 1/3 middle region of the right front hip sensor in the long axis direction.

22. The wearable apparatus of any one of claims 1-21, further comprising a processing circuit, configured to generate a parameter reflecting a joint movement, wherein each of the plurality of inductive sensors is connected with the processing circuit through the conductive wire.

23. The wearable apparatus of any one of claims 1-21, further comprising a processing circuit and distributed readout subsystems, wherein the distributed readout subsystems are configured to read data from at least a portion of the plurality of inductive sensors, and the processing circuit is configured to generate a parameter reflecting a joint movement based on the data, wherein

   at least the portion of the plurality of inductive sensors transmit the data to a specific readout subsystem of the distributed readout subsystem, and
   the distributed readout subsystems are connected with the processing circuit for communication.

24. The wearable apparatus of any one of claims 1-23, wherein the conductive wire includes an elastic and stretchable conductive yarn, and the elastic and stretchable conductive yarn is fixed by weaving.

25. The wearable apparatus of any one of claims 1-24, wherein the conductive wire forms a spiral inductive structure.

26. The wearable apparatus of claim 25, wherein the spiral inductive structure includes more than one turn of the conductive wire.

27. The wearable apparatus of claim 25, wherein a thickness of the conductive wire in a direction perpendicular to a surface of an inductive pattern is not greater than 3 mm.

28. The wearable apparatus of any one of claims 1-27, wherein a surface of the conductive wire is wrapped with at least one layer of insulating material.

29. The wearable apparatus of any one of claims 1-28, wherein a resistance of the inductive structure is less than 100 Ω.

30. The wearable apparatus of claim 25, wherein at least one of the plurality of inductive sensors further includes a substrate configured to carry the spiral inductive structure, and the spiral inductive structure includes at least a first layer coil and a second layer coil; wherein

   the first layer coil and the second layer coil are disposed in layers in a direction perpendicular to the substrate; and
   a current direction in the first layer coil and a current direction in the second layer coil are the same.

31. The wearable apparatus of claim 30, wherein the first layer coil and the second layer coil are respectively disposed on two sides of the substrate.

32. The wearable apparatus of claim 31, wherein a through hole is provided in the substrate, and the first layer coil and the second layer coil are respectively formed by a same conductive wire passing through the through hole.

33. The wearable apparatus of claim 25, wherein at least one of the plurality of inductive sensors further includes a protective layer configured to package the spiral inductive structure.

34. An inductive sensor, comprising:

   a spiral inductive structure formed by a conductive wire; and
   a substrate configured to carry the spiral inductive structure, wherein
   the spiral inductive structure includes at least a first layer coil and a second layer coil;
   the first layer coil and the second layer coil are disposed in layers in a direction perpendicular to the substrate, and
   a current direction in the first layer coil and a current direction in the second layer coil are the same.

35. The inductive sensor of claim 34, wherein the conductive wire includes an elastic and stretchable conductive yarn, and the elastic and stretchable conductive yarn is fixed by weaving.

36. The inductive sensor of claim 34, wherein the spiral inductive structure includes more than one turn of the conductive wire.

37. The inductive sensor of claim 34, wherein a thickness of the conductive wire in a direction perpendicular to a surface of an inductive pattern is not greater than 3 mm.

38. The inductive sensor of claim 34 , wherein a surface of the conductive wire is wrapped with at least one layer of insulating material.

39. The inductive sensor of claim 34, wherein a resistance of the inductive structure is less than 100 $\Omega$.

40. The inductive sensor of claim 34, wherein the first layer coil and the second layer coil are respectively disposed on both sides of the substrate.

41. The inductive sensor of claim 34, wherein a through hole is disposed in the substrate, and the first layer coil and the second layer coil are respectively formed by a same wire passing through the through hole.

42. The inductive sensor of claim 34, further comprising a protective layer configured to package the spiral inductive structure.

43. The inductive sensor of any one of claims 34-42, wherein the inductive sensor is attached to a position of a wearable body corresponding to a joint position and crosses a joint contour, and the inductive sensor is configured to generate variable inductances with deformations of the joint position.

44. The inductive sensor of claim 43, wherein the inductive structure includes a long axis direction and a short axis direction, and the joint contour passes through a 1/3 middle region the inductive structure in the long axis direction.

45. The inductive sensor of claim 43, wherein the inductive sensor crosses an arm root contour and is configured to generate variable inductances with movements of a shoulder joint of a user.

46. The inductive sensor of claim 45, wherein the arm root contour passes through a 1/3 middle region of the inductive structure in the long axis direction.

47. The inductive sensor of claim 43, wherein the inductive sensor crosses an elbow contour and is configured to generate variable inductances with movements of an elbow joint of a user.

48. The inductive sensor of claim 47, wherein the elbow contour passes through a 1/3 middle region of the inductive structure in the long axis direction.

49. The inductive sensor of claim 43, wherein the inductive sensor crosses a waist contour and is configured to generate variable inductances with movements of a waist of a user.

50. The inductive sensor of claim 49, wherein the waist contour passes through a 1/3 middle region of the inductive structure in the long axis direction.

51. The inductive sensor of claim 43, wherein the inductive sensor crosses a knee contour and is configured to generate variable inductances with movements of a knee joint of a user.

52. The inductive sensor of claim 51, wherein the knee contour passes through a 1/3 middle region of the inductive structure in the long axis direction.

53. The inductive sensor of claim 43, wherein the inductive sensor is disposed at a hip bone position and is configured to generate variable inductances with movements of a hip joint of a user.

**FIG. 1**

Front side

**FIG. 2**

Back side

**FIG. 3**

**FIG. 4**

**FIG. 5**

110

Front side

**FIG. 6**

110

232  231

242  241

262  261

663

412  411

420

432  431

Back side

FIG. 7

**FIG. 8**

**FIG. 9**

FIG. 10

1100

FIG. 11

**100**

FIG. 12

1310

1310

1320

1320

1330

1330

FIG. 13

FIG. 14

## INTERNATIONAL SEARCH REPORT

| International application No. |
|---|
| **PCT/CN2023/107984** |

### A. CLASSIFICATION OF SUBJECT MATTER

G06F 3/01(2006.01)i

According to International Patent Classification (IPC) or to both national classification and IPC

### B. FIELDS SEARCHED

Minimum documentation searched (classification system followed by classification symbols)

IPC:G06F

Documentation searched other than minimum documentation to the extent that such documents are included in the fields searched

Electronic data base consulted during the international search (name of data base and, where practicable, search terms used)

CNABS, DWPI, CNTXT, ENTXT, ENTXTC, CNKI: 可穿戴, 电感, 传感器, 关节, 线圈, wearable, inductor, sensor, joint, coil

### C. DOCUMENTS CONSIDERED TO BE RELEVANT

| Category* | Citation of document, with indication, where appropriate, of the relevant passages | Relevant to claim No. |
|---|---|---|
| X | CN 114053097 A (SHANGHAI GREN TECHNOLOGY CO., LTD.) 18 February 2022 (2022-02-18) <br> description, paragraphs [0068]-[0119] | 1-53 |
| A | CN 211720535 U (GOERTEK TECHNOLOGY CO., LTD.) 20 October 2020 (2020-10-20) <br> entire document | 1-53 |
| A | EP 3366211 A1 (KONINKLIJKE PHILIPS N.V.) 29 August 2018 (2018-08-29) <br> entire document | 1-53 |

☐ Further documents are listed in the continuation of Box C.　　☑ See patent family annex.

| * | Special categories of cited documents: | | "T" | later document published after the international filing date or priority date and not in conflict with the application but cited to understand the principle or theory underlying the invention |
|---|---|---|---|---|
| "A" | document defining the general state of the art which is not considered to be of particular relevance | | | |
| "D" | document cited by the applicant in the international application | | "X" | document of particular relevance; the claimed invention cannot be considered novel or cannot be considered to involve an inventive step when the document is taken alone |
| "E" | earlier application or patent but published on or after the international filing date | | | |
| "L" | document which may throw doubts on priority claim(s) or which is cited to establish the publication date of another citation or other special reason (as specified) | | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | | |
| "P" | document published prior to the international filing date but later than the priority date claimed | | "&" | document member of the same patent family |

| Date of the actual completion of the international search | Date of mailing of the international search report |
|---|---|
| **23 October 2023** | **30 October 2023** |

| Name and mailing address of the ISA/CN | Authorized officer |
|---|---|
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/107984**

| Patent document cited in search report | | | Publication date (day/month/year) | Patent family member(s) | Publication date (day/month/year) |
|---|---|---|---|---|---|
| CN | 114053097 | A | 18 February 2022 | None | |
| CN | 211720535 | U | 20 October 2020 | None | |
| EP | 3366211 | A1 | 29 August 2018 | None | |

Form PCT/ISA/210 (patent family annex) (July 2022)

**REFERENCES CITED IN THE DESCRIPTION**

**Patent documents cited in the description**

- CN 2023080488 W

- CN 202210873140